(19) Europäisches Patentamt
European Patent Office
Office européen des brevets

(11) EP 1 891 241 B1

(12) **EUROPEAN PATENT SPECIFICATION**

(45) Date of publication and mention of the grant of the patent:
**12.01.2011 Bulletin 2011/02**

(21) Application number: **06777334.1**

(22) Date of filing: **14.06.2006**

(51) Int Cl.:
***C12Q 1/68*** *(2006.01)*

(86) International application number:
**PCT/EP2006/063215**

(87) International publication number:
**WO 2006/134128 (21.12.2006 Gazette 2006/51)**

(54) **DIAGNOSTIC AND THERAPEUTIC TARGET ADARB2 PROTEINS FOR NEURODEGENERATIVE DISEASES**

ADARB2 PROTEINE ALS DIAGNOSTISCHE UND THERAPEUTISCHE TARGETS FÜR NEURODEGENERATIVE ERKRANKUNGEN

MÉTHODES FAISANT APPEL À DES PROTÉINES ADARB2 CIBLES POUR DIAGNOSTIQUER ET POUR PRONOSTIQUER DES MALADIES NEURODÉGÉNÉRATIVES

(84) Designated Contracting States:
**AT BE BG CH CY CZ DE DK EE ES FI FR GB GR HU IE IS IT LI LT LU LV MC NL PL PT RO SE SI SK TR**

(30) Priority: **16.06.2005 EP 05105316**
**16.06.2005 US 690862 P**

(43) Date of publication of application:
**27.02.2008 Bulletin 2008/09**

(73) Proprietor: **EVOTEC Neurosciences GmbH**
**22525 Hamburg (DE)**

(72) Inventors:
- **POHLNER, Johannes**
**22175 Hamburg (DE)**
- **VON DER KAMMER, Heinz**
**22607 Hamburg (DE)**

(74) Representative: **von Kreisler Selting Werner**
**Deichmannhaus am Dom**
**Bahnhofsvorplatz 1**
**50667 Köln (DE)**

(56) References cited:
**US-A- 5 763 174**

- **DATABASE UniProt [Online] 1 February 2005 (2005-02-01), "Adenosine deaminase, RNA-specific, B2 (RED2 homolog rat)." XP002355598 retrieved from EBI accession no. UNIPROT: Q5VUT6 Database accession no. Q5VUT6**
- **DATABASE UniProt [Online] 7 December 2004 (2004-12-07), "Adenosine deaminase RNA-specific, B2, (RED2 homolog rat)" XP002405378 retrieved from EBI accession no. UNIPROT: Q5VW43_HUMAN Database accession no. Q5VW43_HUMAN**
- **DATABASE UniProt [Online] 10 October 2003 (2003-10-10), "Double-stranded RNA-specific editase B2 (EC 3.5.-.-) (dsRNA adenosine deaminase B2) (RNA dependent adenosine deaminase 3) (RNA editing deaminase 2) (RNA editing enzyme 2)." XP002355599 retrieved from EBI accession no. UNIPROT:RED2_HUMAN Database accession no. RED2_HUMAN**
- **BARBON ALESSANDRO ET AL: "Glutamate receptor RNA editing: A molecular analysis of GluR2, GluR5 and GluR6 in human brain tissues and in NT2 cells following in vitro neural differentiation." MOLECULAR BRAIN RESEARCH, vol. 117, no. 2, October 2003 (2003-10), pages 168-178, XP002355586 ISSN: 0169-328X**
- **KAWAHARA YUKIO ET AL: "Low editing efficiency of GluR2 mRNA is associated with a low relative abundance of ADAR2 mRNA in white matter of normal human brain." EUROPEAN JOURNAL OF NEUROSCIENCE, vol. 18, no. 1, July 2003 (2003-07), pages 23-33, XP002355587 ISSN: 0953-816X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 891 241 B1

- KOEHR GEORG ET AL: "Candidate editases for GluR channels in single neurons of rat hippocampus and cerebellum" NEUROPHARMACOLOGY, vol. 37, no. 10-11, October 1998 (1998-10), pages 1411-1417, XP002355588 ISSN: 0028-3908
- MITTAZ LAUREANE ET AL: "Localization of a novel human RNA-editing deaminase (hRED2 or ADARB2) to chromosome 10p15" HUMAN GENETICS, vol. 100, no. 3-4, 1997, pages 398-400, XP002355589 ISSN: 0340-6717
- MELCHER THORSTEN ET AL: "RED2, a brain-specific member of the RNA-specific adenosine deaminase family" JOURNAL OF BIOLOGICAL CHEMISTRY, vol. 271, no. 50, 1996, pages 31795-31798, XP002355590 ISSN: 0021-9258

## Description

The present invention relates to methods of diagnosing, prognosticating and monitoring the progression of neurodegenerative diseases in a subject. Furthermore, methods of therapy control and screening for modulating agents of neurodegenerative diseases are provided. The invention also discloses pharmaceutical compositions, kits, and recombinant animal models.

Neurodegenerative diseases, in particular Alzheimer's disease (AD), have a strongly debilitating impact on a patient's life. Furthermore, these diseases constitute an enormous health, social, and economic burden. AD is the most common neurodegenerative disease, accounting for about 70 % of all dementia cases, and it is probably the most devastating age-related neurodegenerative condition affecting about 10 % of the population over 65 years of age and up to 45 % over age 85 (Vickers et al., Progress in Neurobiology 2000, 60: 139-165; Walsh and Selkoe, Neuron 2004, 44:181-193). Presently, this amounts to an estimated 12 million cases in the US, Europe, and Japan. This situation will inevitably worsen with the demographic increase in the number of old people in developed countries. The neuropathological hallmarks that occur in the brains of individuals with AD are senile plaques, composed of amyloid-β protein, and profound cytoskeletal changes coinciding with the appearance of abnormal filamentous structures and the formation of neurofibrillary tangles.

The amyloid-β protein evolves from the cleavage of the amyloid precursor protein (APP) by different kinds of proteases (Selkoe and Kopan, Annu Rev Neurosci 2003, 26:565-597; Ling et al., Int J Biochem Cell Biol 2003, 35: 1505-1535). Two types of plaques, diffuse plaques and neuritic plaques can be detected in the brain of AD patients. They are primarily found in the cerebral cortex and hippocampus. The generation of toxic Aß deposits in the brain starts very early in the course of AD, and it is discussed to be a key player for the subsequent destructive processes leading to AD pathology. The other pathological hallmarks of AD are neurofibrillary tangles (NFTs) and abnormal neurites, described as neuropil threads (Braak and Braak, J Neural Transm 1998, 53: 127-140). NFTs emerge inside neurons and consist of chemically altered tau, which forms paired helical filaments (PHF) twisted around each other. Along the formation of NFTs, a loss of neurons can be observed (Johnson and Jenkins, J Alzheimers Dis 1996, 1: 38-58; Johnson and Hartigan, J Alzheimers Dis 1999, 1: 329-351). The appearance of neurofibrillary tangles and their increasing number correlates well with the clinical severity of AD (Schmitt et al., Neurology 2000, 55: 370-376). AD is a progressive disease that is associated with early deficits in memory formation and ultimately leads to the complete erosion of higher cognitive function. The cognitive disturbances include among other things memory impairment, aphasia, agnosia and the loss of executive functioning. A characteristic feature of the pathogenesis of AD is the selective vulnerability of particular brain regions and subpopulations of nerve cells to the degenerative process. Specifically, the inferior temporal lobe region and the hippocampus are affected early and more severely during the progression of the disease. On the other hand, neurons within the frontal cortex, occipital cortex, and the cerebellum remain largely intact and are protected from neurodegeneration (Terry et al., Annals of Neurology 1981, 10: 184-92).

US 5,763,174 discloses human polynucleotide sequences and the recombinant human DRADA proteins encoded thereby and methods of use thereof.

Further, DATABASE UniProt [Online] 1 February 2005 discloses adenosine deaminase, RNA-specific, B2 (RED2 homolog rat) and DATABASE UniProt [Online] 7 December 2004 discloses adenosine deaminase, RNA-specific, B2 (RED2 homolog rat), too.

Currently, there is no cure for AD, nor is there an effective treatment to halt the progression of AD or even to diagnose AD ante-mortem with high probability. Several risk factors have been identified that predispose an individual to develop AD, among them most prominently the epsilon 4 allele of the three different existing alleles (epsilon 2, 3, and 4) of the apolipoprotein E gene (ApoE) (Strittmatter et al., Proc Natl Acad Sci USA 1993, 90: 1977-81; Roses, Ann NY Acad Sci 1998, 855: 738-43). Although there are rare examples of early-onset AD which have been attributed to genetic defects in the genes for amyloid precursor protein (APP) on chromosome 21, presenilin-1 on chromosome 14, and presenilin-2 on chromosome 1, the prevalent form of late-onset sporadic AD is of hitherto unknown etiologic origin. The late onset and complex pathogenesis of neurodegenerative disorders pose a formidable challenge to the development of therapeutic and diagnostic agents. It is crucial to expand the pool of potential drug targets and diagnostic markers.

It is therefore an object of the present invention to provide insight into the pathogenesis of neurological diseases and to provide methods, materials, agents, compositions, and animal models which are suited inter alia for the diagnosis and development of a treatment of these diseases. This object has been solved by the features of the independent claims. The subclaims define preferred embodiments of the present invention.

The present invention is based on the detection of dysregulated, differential expression of a gene of the ADAR gene family, coding for an adenosine deaminase, the RNA-specific adenosine deaminase ADARB2, also named ADAR3 or RED2, and of the protein products of ADARB2 in human Alzheimer's disease brain samples. ADARB2 has been formerly known as ADARB; such formerly used gene symbol ADARB has been exchanged to ADARB2 in accordance with the HUGO (Human Genome Organisation) gene nomenclature (http://www.gene.ucl.ac.uk/nomenclature/). Nuclear pre-mRNA editing by selective adenosine deamination occurs in all organisms. This posttranscriptional mechanism can alter codons and hence the structure and function of proteins. Two of the mammalian double-stranded (ds) RNA-specific adenosine deaminases, ADAR1 and ADAR2 have been found to convert adenosine residues into inosines in dsRNA, and to be involved in A-to-I editing of transcripts of glutamate receptor subunits (GluR2) and serotonin receptor subtype 2C (5HT2c) (Seeburg, Neuron 2002, 35:17-20; Maas et al., J. Biol. Chem. 2003,278:1391-1394). These RNA editing events regulate functions of GluR2 and 5HT2c through controlling the maturation, intracellular trafficking and assembly with other subunits of these transmembrane proteins. For instance, the unedited form of GluR2 has high Ca2+ permeability, probably resulting in neuronal death (Reenan, Trends in Genetics 2001, 17:53-56). Deficiency of A-to-I RNA editing by ADAR1 or ADAR2 is related to diseases such as epilepsy, ALS, and depression, which is due to underediting of GluR2, 5HT2c and Kv2-potassium channel (Kwak et al., J. Mol. Med. 2005, 83:110-120; Maas et al., J. Biol. Chem. 2003, 278:1391-1394). ADAR2 knockout mice died young after repeated episodes of epileptic seizures (Higuchi et al., Nature 2000, 406:78-81). It has been also reported that lethal phenotypes including dyserythropoietic defects were observed in chimeric mouse embryos derived from ADAR1+/- embryonic stem cells (Wang et al., Science 2000, 290: 1765-1768). ADARB2 (alias ADAR3) is the third member of the ADAR gene family, and contains the common structural features such as a C-terminal deaminase domain and an internal double-stranded RNA binding domain. In addition, it includes an arginine and lysine-rich single-stranded (ss) RNA binding domain at the N-terminus. Thus, ADARB2 is capable of binding not only to dsRNA but also to ssRNA. In contrast to the ubiquitous expressions of ADAR1 and ADAR2, ADARB2 expression is restricted to the brain, where the expression is widespread reaching highest levels in olfactory bulb and thalamus. Moreover, no enzymatic activity of ADARB2 on editing of GluR2 and 5HT2c mRNA has been demonstrated yet through both *in vitro* and *in vivo* assays. Remarkably, ADARB2 display strong competitive inhibitory effect on RNA editing activities of the other ADAR members, at least *in vitro,* suggesting the possibility that ADARB2 may play a role in the regulation of substrate-specific RNA editing in mammalian brains (Melcher et al., J. Biol. Chem. 1996, 271:31795-31798; Chen et al., RNA 2000, 6:755-767).

Figure 1 discloses the identification of differences in the levels of ADARB2 gene derived mRNA in human brain tissue samples from individuals corresponding to different Braak stages as measured and compared by GeneChip analyses. It indicates that the levels of the respective mRNA species correlate quantitatively with AD progression and thus are indicative for AD as measured by the neuropathological staging of brain tissue samples according to Braak and Braak (Braak staging).

Figure 2 lists the data for the verification of differences in the levels of ADARB2 gene derived mRNA in human brain tissue samples from individuals corresponding to different Braak stages indicative for AD as measured by quantitative RT-PCR analysis.

Figure 3 shows the analysis of absolute levels of ADARB2 gene derived mRNA in human brain tissue samples from individuals corresponding to different Braak stages indicative for AD as measured by quantitative RT-PCR and using statistical method of the median at 98%-confidence level.

Figure 4A discloses SEQ ID NO: 1, the amino acid sequence of the human ADARB2 splice variant 1 protein.

Figure 4B discloses SEQ ID NO: 2, the amino acid sequence of the human ADARB2 splice variant 2 protein.

Figure 4C discloses SEQ ID NO: 3, the amino acid sequence of the human ADARB2 splice variant 3 protein.

Figure 4D discloses SEQ ID NO: 4, the amino acid sequence of the human ADARB2 splice variant 4 protein.

Figure 5A shows SEQ ID NO: 5, the nucleotide sequence of the human ADARB2 splice variant 1 cDNA.

Figure 5B shows SEQ ID NO: 6, the nucleotide sequence of the human ADARB2 splice variant 2 cDNA.

Figure 5C shows SEQ ID NO: 7, the nucleotide sequence of the human ADARB2 splice variant 3 cDNA.

Figure 5D shows SEQ ID NO: 8, the nucleotide sequence of the human ADARB2 splice variant 4 cDNA.

Figure 6A depicts SEQ ID NO: 9, the coding sequence (cds) of the human ADARB2 splice variant 1.

Figure 6B depicts SEQ ID NO: 10, the coding sequence (cds) of the human ADARB2 splice variant 2.

Figure 6C depicts SEQ ID NO: 11, the coding sequence (cds) of the human ADARB2 splice variant 3.

Figure 6D depicts SEQ ID NO: 12, the coding sequence (cds) of the human ADARB2 splice variant 4.

Figure 7 depicts the sequence alignment of the primers used for ADARB2 transcription level profiling by quantitative RT-PCR with the corresponding clippings of ADARB2 cDNA.

Figure 8 schematically charts the alignment of the ADARB2 cDNA sequence, the coding sequence and both primer sequences used for ADARB2 transcription level profiling.

Figure 9 shows an immunoblot (Western blot) analysis of the affinity-purified polyclonal rabbit anti- ADARB2 antiserum sc-10014.

Figures 10A exemplifies the increase in the level of ADARB2 protein in brain cerebral cortex tissue samples from AD patients (Braak 4-6) when compared to the levels observed in respective samples from age-matched controls (Braak 0-2) which have not been diagnosed to suffer from AD signs and symptoms.

Figures 10B exemplifies the increase in the level of ADARB2 protein in brain cerebral white matter tissue samples from AD patients (Braak 4 and 6) when compared to the levels observed in respective samples from age-matched controls (Braak 0 and 1) which have not been diagnosed to suffer from AD signs and symptoms.

Figure 11 shows inducible ADARB2 expression in H4-neuroglioma cells stably expressing the Swedish Mutant APP by Western blot analysis.

Figure 12 shows inducible ADARB2 expression in H4-neuroglioma cells stably expressing the Swedish Mutant APP by immunofluorescence analysis.

The singular forms "a", "an", and "the" as used herein and in the claims include plural reference unless the context dictates otherwise. For example, "a cell" means as well a plurality of cells, and so forth.

The term "and/or" as used in the present specification and in the claims implies that the phrases before and after this term are to be considered either as alternatives or in combination. For instance, the wording "determination of a level and/or an activity" means that either only a level, or only an activity, or both a level and an activity are determined.

The term "level" as used herein is meant to comprise a gage of, or a measure of the amount of, or a concentration of a substance such as a transcription product, for instance an mRNA, or a translation product, for instance a protein or polypeptide.

The term "activity" as used herein shall be understood as a measure for the ability of a substance, such as transcription product or a translation product to produce a biological effect or a measure for a level of biologically active molecules. The term "activity" also refers to biological activity and/or pharmacological activity which refer to binding, antagonization, repression, blocking, neutralization or sequestration of a deaminase or deaminase subunit and which refers to activation, agonization, and up-regulation of a deaminase or deaminase subunit.

The terms "level" and/or "activity" as used herein further refer to gene expression levels or gene activity. Gene expression can be defined as the utilization of the information contained in a gene by transcription and translation leading to the production of a gene product.

"Dysregulation" shall mean an up-regulation or down-regulation of gene expression and/or an increase or decrease in the stability of the gene products. A gene product comprises either RNA or protein and is the result of expression of a gene. The amount of a gene product can be used to measure how active a gene is and how stable its gene products are.

The term "gene" as used in the present specification and in the claims comprises both coding regions (exons) as well as non-coding regions (e.g. non-coding regulatory elements such as promoters or enhancers, introns, leader and trailer sequences).

The term "ORF" is an acronym for "open reading frame" and refers to a nucleic acid sequence that does not possess a stop codon in at least one reading frame and therefore can potentially be translated into a sequence of amino acids.

"Regulatory elements" shall comprise inducible and non-inducible promoters, enhancers, operators, and other elements that drive and regulate gene expression.

The term "fragment" as used herein is meant to comprise e.g. an alternatively spliced, or truncated, or otherwise cleaved transcription product or translation product. For example, the proteins having SEQ ID NO: 1, SEQ ID NO: 2, SEQ ID NO: 3, and SEQ ID NO: 4 are translation products of the gene coding for ADARB2 proteins and constitute splice variants.

The term "derivative" as used herein refers to a mutant, or an RNA-edited, or a chemically modified, or otherwise altered transcription product, or to a mutant, or chemically modified, or otherwise altered translation product. For the purpose of clarity, a derivative transcript, for instance, refers to a transcript having alterations in the nucleic acid sequence such as single or multiple nucleotide deletions, insertions, or exchanges. A derivative translation product, for instance, may be generated by processes such as altered phosphorylation, or glycosylation, or acetylation, or lipidation, or by altered signal peptide cleavage or other types of maturation cleavage. These processes may occur post-translationally.

The term "modulator" as used in the present invention and in the claims refers to a molecule capable of changing or altering the level and/or the activity of a gene, or a transcription product of a gene, or a translation product of a gene. A "modulator" refers to a molecule which has the capacity to either enhance or inhibit, thus to "modulate" a functional property of a protein, to "modulate" binding, antagonization, repression, blocking, neutralization or sequestration, activation, agonization and up-regulation. "Modulation" will be also used to refer to the capacity to affect the biological activity of a cell. Preferably, a "modulator" is capable of changing or altering the biological activity of a transcription product or a translation product of a gene. Said modulation, for instance, may be an increase or a decrease in the biological activity and/or pharmacological activity, a change in binding characteristics, or any other change or alteration in the biological, functional, or immunological properties of said translation product of a gene.

The terms "agent", "reagent", or "compound" refer to any substance, chemical, composition, or extract that have a positive or negative biological effect on a cell, tissue, body fluid, or within the context of any biological system, or any assay system examined. They can be agonists, antagonists, partial agonists or inverse agonists of a target. Such agents, reagents, or compounds may be nucleic acids, natural or synthetic peptides or protein complexes, or fusion proteins. They may also be antibodies, organic or anorganic molecules or compositions, small molecules, drugs and any combinations of any of said agents above. They may be used for testing, for diagnostic or for therapeutic purposes.

The terms "oligonucleotide primer" or "primer" refer to short nucleic acid sequences which can anneal to a given target polynucleotide by hybridization of the complementary base pairs and can be extended by a polymerase. They may be chosen to be specific to a particular sequence or they may be randomly selected, e.g. they will prime all possible sequences in a mix. The length of primers used herein may vary from 10 nucleotides to 80 nucleotides. "Probes" are short nucleic acid sequences of the nucleic acid sequences described and disclosed herein or sequences complementary therewith. They may comprise full length sequences, or fragments, derivatives, isoforms, or variants of a given sequence. The identification of hybridization complexes between a "probe" and an assayed sample allows the detection of the presence of other similar sequences within that sample.

As used herein, "homolog or homology" is a term used in the art to describe the relatedness of a nucleotide or peptide sequence to another nucleotide or peptide sequence, which is determined by the degree of identity and/or similarity between said sequences compared.

In the art, the terms "identity" and "similarity" mean the degree of polypeptide or polynucleotide sequence relatedness which are determined by matching a query sequence and other sequences of preferably the same type (nucleic acid or protein sequence) with each other. Preferred computer program methods to calculate and determine "identity" and "similarity" include, but are not limited to GCG BLAST (Basic Local Alignment Search Tool) (Altschul et al., J. Mol. Biol. 1990, 215: 403-410; Altschul et al., Nucleic Acids Res. 1997, 25: 3389-3402; Devereux et al., Nucleic Acids Res. 1984, 12: 387), BLASTN 2.0 (Gish W., http://blast.wustl.edu, 1996-2002), FASTA (Pearson and Lipman, Proc. Natl. Acad. Sci. USA 1988, 85: 2444-2448), and GCG GelMerge which determines and aligns a pair of contigs with the longest overlap (Wilbur and Lipman, SIAM J. Appl. Math. 1984, 44: 557-567; Needleman and Wunsch, J. Mol. Biol. 1970, 48: 443-453).

The term "variant" as used herein refers to any polypeptide or protein, in reference to polypeptides and proteins disclosed in the present invention, in which one or more amino acids are added and/or substituted and/or deleted and/or inserted at the N-terminus, and/or the C-terminus, and/or within the native amino acid sequences of the native polypeptides or proteins of the present invention, but retains its essential properties. Furthermore the term "variant" as used herein refers to any mRNA, in reference to gene transcripts disclosed in the present invention, in which one or more nucleotides are added and/or substituted and/or deleted.

Furthermore, the term "variant" shall include any shorter or longer version of a polypeptide or protein. "Variants" shall also comprise a sequence that has at least about 80 % sequence identity, more preferably at least about 85 % sequence identity, and most preferably at least about 90 % sequence identity over a length of at least 200 amino acids of ADARB2 proteins having SEQ ID NO: 1, or SEQ ID NO: 2, or SEQ ID NO: 3, or SEQ ID NO: 4. "Variants" also include, for example, proteins with conservative amino acid substitutions in highly conservative regions.

Furthermore, the term "variant" shall include any shorter or longer version of a gene transcript. "Variants" shall also comprise a sequence that has at least about 80 % sequence identity, more preferably at least about 85 % sequence identity, and most preferably at least about 90 % sequence identity over a length of at least 600 nucleotides of ADARB2 gene transcripts having SEQ ID NO: 5, or SEQ ID NO: 6, or SEQ ID NO: 7, or SEQ ID NO: 8. Sequence variations shall be included wherein a codon is replaced with another codon due to alternative base sequences, but the amino acid sequence translated by the DNA sequence remains unchanged. This known in the art phenomenon is called redundancy of the set of codons which translate specific amino acids.

"Proteins and polypeptides" of the present invention include variants, fragments and chemical derivatives of the proteins comprising the amino acid sequences of ADARB2 proteins having SEQ ID NO: 1, or SEQ ID NO:2, or SEQ ID NO:3, or SEQ ID NO:4. Included shall be such exchange of amino acids which would have no effect on functionality, such as arginine for lysine, valine for leucine, asparagine for glutamine. Proteins and polypeptides can be included which can be isolated from nature or be produced by recombinant and/or synthetic means. Native proteins or polypeptides refer to naturally-occurring truncated or secreted forms, naturally occurring variant forms (e.g. splice-variants) and naturally occurring allelic variants.

The term "isolated" as used herein is considered to refer to molecules or substances which have been changed and/or that are removed from their natural environment, i.e. isolated from a cell or from a living organism in which they normally occur, and that are separated or essentially purified from the coexisting components with which they are found to be associated in nature. This notion further means that the sequences encoding such molecules can be linked by the hand of man to polynucleotides, to which they are not linked in their natural state and such molecules can be produced by recombinant and/or synthetic means, it is also said that they are "non-native". Even if for said purposes those sequences may be introduced into living or non-living organisms by methods known to those skilled in the art, and even if those sequences are still present in said organisms, they are still considered to be isolated. In the present invention, the terms "risk", "susceptibility", and "predisposition" are tantamount and are used with respect to the probability of developing a neurodegenerative disease, preferably Alzheimer's disease.

The term "AD" shall mean Alzheimer's disease. "AD-type neuropathology", "AD pathology" as used herein refers to neuropathological, neurophysiological, histopathological and clinical hallmarks, signs and symptoms as described in the instant invention and as commonly known from state-of-the-art literature (see: Iqbal, Swaab, Winblad and Wisniewski, Alzheimer's Disease and Related Disorders (Etiology, Pathogenesis and Therapeutics), Wiley & Sons, New York, Weinheim, Toronto, 1999; Scinto and Daffner, Early Diagnosis of Alzheimer's Disease, Humana Press, Totowa, New Jersey, 2000; Mayeux and Christen, Epidemiology of Alzheimer's Disease: From Gene to Prevention, Springer Press, Berlin, Heidelberg, New York, 1999; Younkin, Tanzi and Christen, Presenilins and Alzheimer's Disease, Springer Press, Berlin, Heidelberg, New York, 1998).

The term "Braak stage" or "Braak staging" refers to the classification of brains according to the criteria proposed by Braak and Braak (Braak and Braak, Acta Neuropathology 1991, 82: 239-259). Braak staging of AD rates the extent and distribution of neurofibrillary pathology in determined regions of the forebrain and divides the neuropathologic progression of AD into six stages (stage 0 to 6). It is a well established and universally accepted procedure in post-mortem neuropathological staging of AD. It has convincingly been shown that there is a significant correlation between an AD patient's clinical condition with respect to mental status and cognitive function/impairment and the corresponding Braak stage obtained after autopsy (Bancher et al., Neuroscience Letters 1993, 162:179-182; Gold et al., Acta Neuropathol 2000, 99: 579-582). Likewise, a correlation between neurofibrillary changes and neuronal cellular pathology has been found (R6ssler et al., Acta Neuropathol 2002, 103:363-369), and both have been reported to predict cognitive function (Giannakopoulos et al., Neurology 2003, 60:1495-1500; Bennett et al., Arch Neurol 2004, 61:378-384). Moreover, a pathogenic cascade has been proposed that involves the deposition of beta-amyloid peptide and finally cumulates in the formation of neurofibrillary tangles, the latter thus witnessing the precedence of earlier AD-specific events at the molecular/cellular level (Metsaars et al., Neurobiol Aging 2003, 24:563-572).

In the instant invention, Braak stages are therefore used as a surrogate marker of disease progression independent of the clinical presentation/condition of the individual donor, i.e. independent of the presence or absence of reported mental illness, cognitive deficits, decline in other neuropsychiatric parameters, or the overt clinical diagnosis of AD. I.e. it is presumed that the neurofibrillary changes on which the Braak staging reflect the underlying molecular and cellular pathomechanisms in general and hence define a (pre-)morbid condition of the brain, meaning that e.g. a donor staged Braak 1 represents by definition an earlier stage of molecular/cellular pathogenesis than a donor staged 2 (or higher), and that therefore a donor of Braak stage 1 can e.g. be regarded as a control individual when compared to donors of any higher Braak stage. In this regard, the differentiation between control individual and affected individual may not necessarily be the same as the clinical diagnosis based differentiation between healthy control donor and AD patient, but it rather refers to a presumed difference in the (pre-) morbid status as deduced from and mirrored by a surrogate marker, the Braak stage.

In the instant invention Braak stage 0 may represent persons which are not considered to suffer from Alzheimer's disease signs and symptoms, and Braak stages 1 to 4 may represent either healthy control individuals or AD patients depending on whether said individuals were suffering already from clinical signs and symptoms of AD. The higher the Braak stage the more likely is the possibility to display signs and symptoms of AD or the risk to develop signs and symptoms of AD. For a neuropathological assessment, i.e. an estimation of the probability that pathological changes of AD are the underlying cause of dementia, a recommendation is given by Braak H. (www. alzforum.org).

The values obtained from controls are the reference values representing a known health status and the values obtained from patients are the reference values representing a known disease status.

Neurodegenerative diseases or disorders according to the present invention comprise Alzheimer's disease, Parkinson's disease, Huntington's disease, amyotrophic lateral sclerosis, Pick's disease, fronto-temporal dementia, progressive nuclear palsy, corticobasal degeneration, cerebro-vascular dementia, multiple system atrophy, argyrophilic grain dementia and other tauopathies, and mild-cognitive impairment. Further conditions involving neurodegenerative processes are, for instance, ischemic stroke, age-related macular degeneration, narcolepsy, motor neuron diseases, prion diseases, traumatic nerve injury and repair, and multiple sclerosis.

The present invention discloses the identification, the differential expression, the differential regulation, a dysregulation of a gene of the of the ADAR gene family coding for the RNA-specific adenosine deaminase ADARB2, also named ADAR3 or RED2 or simply ADARB, and of the protein products of said gene ADARB2 (ADARB), in specific samples, in specific brain regions of AD patients, in specific brain regions of individuals grouped into different Braak stages, in comparison with each other and/or in comparison to age-matched control individuals. The present invention discloses that the gene expression for ADARB2 (ADARB) is varied, is dysregulated in brains of AD patients as compared to the respective brain regions of control individuals, in that ADARB2 mRNA levels are increased, are up-regulated in the inferior temporal cortex and in the frontal cortex of AD patients. Further, the present invention discloses that the ADARB2 expression differs in specific brain regions of individuals grouped into different Braak stages with an increase in expression level starting already at early Braak stages (Braak 1-3) and with a progressive increase with the course of late Braak stages (Braak 4-6) predominantly in the inferior temporal cortex.

The differences observed at the ADARB2 gene transcriptional level, when compared between AD patients and control individuals but also between the different Braak stages, are further supported by substantial differences that can be found at the ADARB2 protein level. In comparison to the control individuals, in brain specimens from AD patients the levels of ADARB2 protein are increased substantially. This dysregulation of the ADARB2 gene expression and the changes in levels of the corresponding gene products which parallels the development of AD-type pathology clearly reflects a link between ADARB2 and AD and is indicative for the progressive pathological events in the course of the disease. To date, no experiments have been described that demonstrate a relationship between the dysregulation of ADARB2 gene expression and the pathology of neurodegenerative diseases, in particular AD. Likewise, no mutations in the ADARB2 gene have been described to be associated with said diseases. Linking the ADARB2 gene to such diseases offers new ways, inter alia, for the diagnosis and treatment of said diseases. Additionally, linking ADARB2 to pathological events occurring already early in the course of AD provides the possibility of a treatment which will prevent the initiation of AD pathology, a treatment which will be applied before non-repairable damages of the brain occur. Consequently, the present invention has utility for diagnostic evaluation, for diagnostic monitoring of persons undergoing a treatment, for prognosis as well as for the identification of a predisposition to a neurodegenerative disease, in particular AD.

The present invention discloses a dysregulation of a gene coding for ADARB2 and of its gene products in specific brain regions of AD patients. Neurons within the inferior temporal lobe, the entorhinal cortex, the hippocampus, and the amygdala are subject to degenerative processes in AD (Terry et al., Annals of Neurology 1981, 10:184-192). These brain regions are mostly involved in the processing of learning and memory functions and display a selective vulnerability to neuronal loss and degeneration in AD. In contrast, neurons within the frontal cortex, the occipital cortex, and the cerebellum remain largely intact and preserved from neurodegenerative processes. Brain tissues from the frontal cortex (F) and the inferior temporal cortex (T) of AD patients and of age-matched controls were used for the herein disclosed examples. Consequently, the ADARB2 gene and its corresponding transcription and/or translation products play a causative role, and/or have an influence on the selective neuronal degeneration and/or neuroprotection.

In one aspect, the invention features a method of diagnosing or prognosticating Alzheimer's disease in a subject, or of determining whether a subject has a predisposition of developing said disease. The method comprises: determining a level of (i) a transcription product of a gene coding for ADARB2 proteins, and/or of (ii) a translation product of a gene coding for ADARB2 proteins, and/or of (iii) a fragment of said transcription or translation product in a sample obtained from said subject and comparing said level of said transcription product and/or said translation product and/or said fragment thereof to a reference value representing a disease status of Alzheimer's disease (patient) and/or to a reference value representing a known health status (control), and/or to a reference value representing a known Braak stage and analysing whether said level is increased compared to a reference value representing a known health status, and/or is similar or equal to a reference value representing a disease status of Alzheimer's disease and/or is similar or equal compared to a reference value representing a known Braak stage which is an indication that said subject has Alzheimer's disease, or that said subject is at increased risk of developing Alzheimer's disease. The wording "in a subject" refers to results of the methods disclosed as far as they relate to a disease afflicting a subject, that is to say, said disease being "in" a subject.

In a further aspect, the invention features a method of monitoring the progression of a neurodegenerative disease in a subject. A level, expression or an activity, or both said level, expression and said activity, of (i) a transcription product of a gene coding for ADARB2 proteins, and/or of (ii) a translation product of a gene coding for ADARB2 proteins, and/or of (iii) a fragment, or derivative, or variant of said transcription or translation product in a sample obtained from said subject is determined. Said level, expression and/or said activity are compared to a reference value representing a known disease or health status or a known Braak stage. Thereby, the progression of said Alzheimer's disease in said subject is monitored.

Disclosed is a method of evaluating a treatment or monitoring the effect of a treatment for a neurodegenerative disease, comprising determining a level, expression or an activity, or both said level, expression and said activity of (i) a transcription product of a gene coding for ADARB2 proteins, and/or of (ii) a translation product of a gene coding for ADARB2 proteins, and/or of (iii) a fragment, or derivative, or variant of said transcription or translation product in a sample obtained from a subject being treated for said disease. Said level, expression or said activity, or both said level, expression and said activity are compared to a reference value representing a known disease or health status or a known Braak stage, thereby evaluating the treatment for said neurodegenerative disease.

In a preferred embodiment, the level, expression of (i) a transcription product of a gene coding for ADARB2 proteins, and/or of (ii) a translation product of a gene coding ADARB2 proteins, and/or of (iii) a fragment of said transcription or translation product in a series of samples taken from said subject over a period of time is compared, in order to monitor the progression of said disease. In further preferred embodiments, said subject receives a treatment prior to one or more of said sample gatherings. In yet another preferred embodiment, said level is determined before and after said treatment of said subject.

It is preferred that said level, the expression of said transcription product and/or said translation product of ADARB2 and of its fragments is increased, is up-regulated in samples obtained from AD patients as compared to samples obtained from persons not suffering from AD, control persons. For example, the expression of the transcription product and/or the translation product of ADARB2 and of its fragments is measured from samples of patients and compared with the expression of the transcription product and/or the translation product of ADARB2 and of its fragments in a sample of a healthy control subject (reference sample).

In a preferred embodiment of the herein claimed methods, kits, recombinant animals, molecules, assays, and uses of the instant invention, said ADARB2 gene codes for proteins having SEQ ID NO: 1 (splice variant 1 (sv1), UniProt primary accession number Q9NS39), or SEQ ID NO: 2 (splice variant 2 (sv2), UniProt primary accession number Q5VW42), or SEQ ID NO:3 (splice variant 3 (sv3), UniProt primary accession number Q5VW43), or SEQ ID NO:4 (splice variant 4 (sv4), UniProt primary accession number Q86X17). The amino acid sequences of said splice variants are deduced from the mRNA sequences of SEQ ID NO: 5 which correspond to the cDNA sequence of Ensembl ID ENST00000381312, of SEQ ID NO: 6 which correspond to the cDNA sequence of Ensembl ID ENST00000381310, of SEQ ID NO: 7 which correspond to the cDNA sequence of Ensembl ID ENST00000381305, and of SEQ ID NO: 8 which correspond to the cDNA sequence of Ensembl ID ENST00000337857, respectively. In the instant invention ADARB2 also refers to the nucleic acid sequences SEQ ID NO: 9, SEQ ID NO: 10, SEQ ID NO: 11, and SEQ ID NO: 12, representing the coding sequences (cds) of human ADARB2 splice variants. In the instant invention said sequences are "isolated" as the term is employed herein. Further, in the instant invention, the gene coding for said ADARB2 proteins (splice variants sv1, sv2, sv3, and sv4) is also generally referred to as the ADARB2 gene or simply ADARB. The proteins (in particular splice variants sv1, sv2, sv3, and sv4) of ADARB2 are also generally referred to as the ADARB2 proteins, ADARB2 splice variants or simply ADARB2.

In a further preferred embodiment of the herein claimed methods, kits, recombinant animals, molecules, assays, and uses of the instant invention, said neurodegenerative disease or disorder is Alzheimer's disease, and said subjects suffer from signs and symptoms of Alzheimer's disease.

It is preferred that the sample to be analyzed and determined is selected from the group comprising brain tissue or other tissues, or body cells. The sample can also comprise cerebrospinal fluid or other body fluids including saliva, urine, stool, blood, serum plasma, or mucus. Preferably, the methods of diagnosis, prognosis, monitoring the progression or evaluating a treatment for Alzheimer's disease, according to the instant invention, can be practiced *ex corpore,* and such methods preferably relate to samples, for instance, body fluids or cells, removed, collected, or isolated from a subject or patient or a control person.

In further preferred embodiments, said reference value is that of a level, of expression of (i) a transcription product of the gene coding for ADARB2 proteins, and/or of (ii) a translation product of the gene coding for ADARB2 proteins, and/or of (iii) a fragment of said transcription or translation product in a sample obtained from a subject not suffering from said Alzheimer's disease (control sample, control, healthy control person) or in a sample obtained from a subject suffering from Alzheimer's disease (patient sample, patient, AD sample) or from a person with a defined Braak stage which may suffer or may not suffer from signs and symptoms of AD.

In preferred embodiments, an alteration in the level and/or expression of a transcription product of the gene coding for ADARB2 proteins and/or of a translation product of the gene coding for ADARB2 proteins and/or of a fragment thereof in a sample cell, or tissue, or body fluid taken from said subject relative to a reference value representing a known health status (control sample) indicates a diagnosis, or prognosis, or increased risk of becoming diseased with AD.

In a further preferred embodiment, an equal or similar level and/or expression of a transcription product of the gene coding for ADARB2 proteins and/or of a translation product of the gene coding for ADARB2 proteins and/or of a fragment thereof in a sample cell, or tissue, or body fluid obtained from a subject relative to a reference value representing a known disease status of Alzheimer's disease (AD patient sample), indicates a diagnosis, or prognosis, or increased risk of becoming diseased with said Alzheimer's disease.

In another further preferred embodiment, an equal or similar level, expression of a transcription product of the gene coding for ADARB2 proteins and/or of a translation product of the gene coding for ADARB2 proteins and/or of a fragment thereof in a sample cell, or tissue, or body fluid obtained from a subject relative to a reference value representing a known Braak stage which Braak stage reflects a high risk of developing signs and symptoms of AD, indicates a diagnosis, or prognosis, or an increased risk of becoming diseased with AD.

It is preferred however that said varied, altered level, altered expression of said transcription product and/or said translation product of ADARB2 and of its fragments is an increase, an up-regulation.

In preferred embodiments, measurement of the level of transcription products and/or of expression of the gene coding for ADARB2 proteins is performed in a sample obtained from a subject using a quantitative PCR-analysis with primer. combinations to amplify said gene specific sequences from cDNA obtained by reverse transcription of RNA extracted from a sample of a subject. Primer combinations (SEQ ID NO: 13, SEQ ID NO: 14) are given in Example (vi) of the instant invention, but also other primers generated from the sequences as disclosed in the instant invention can be used. A Northern blot or a ribonuclease protection assay (RPA) with probes specific for said gene can also be applied. It might further be preferred to measure transcription products by means of chip-based microarray technologies. These techniques are known to those of ordinary skill in the art (see Sambrook and Russell, Molecular Cloning: A Laboratory Manual, Cold Spring Harbor Laboratory Press, Cold Spring Harbor, New York, 2001; Schena M., Microarray Biochip Technology, Eaton Publishing, Natick, MA, 2000). An example of an immunoassay is the detection and measurement of enzyme activity as disclosed and described in the patent application WO02/14543.

The invention also relates to the construction and the use of primers and probes which are unique to the nucleic acid sequences, or fragments, or variants thereof, as disclosed in the present invention. The oligonucleotide primers and/or probes can be labeled specifically with fluorescent, bioluminescent, magnetic, or radioactive substances. The invention further relates to the detection and the production of said nucleic acid sequences, or fragments and variants thereof, using said specific oligonucleotide primers in appropriate combinations. PCR-analysis, a method well known to those skilled in the art, can be performed with said primer combinations to amplify said gene specific nucleic acid sequences from a sample containing nucleic acids. Such sample may be derived either from healthy or diseased subjects or subjects with defined Braak stages. Whether an amplification results in a specific nucleic acid product or not, and whether a fragment of different length can be obtained or not, may be indicative for a neurodegenerative disease, in particular Alzheimer's disease. Thus, the invention provides nucleic acid sequences, oligonucleotide primers, and probes of at least 10 bases in length up to the entire coding and gene sequences, useful for the detection of gene mutations and single nucleotide polymorphisms in a given sample comprising nucleic acid sequences to be examined, which may be associated with neurodegenerative diseases, in particular Alzheimer's disease. This feature has utility for developing rapid DNA-based diagnostic tests, preferably also in the format of a kit. Primers for ADARB2 are exemplarily described in Example (vi).

Furthermore, a level and/or expression of a translation product of the gene coding for ADARB2 proteins and/or of a fragment of said translation product, and/or the level of said translation product, and/or of a fragment thereof, can be detected using an immunoassay, an activity assay, e.g. a cellular deaminase assay, a cellular assay measuring GluR2 and 5HT2c activity, an assay measuring RNA interaction, and/or a binding assay. These assays can measure the amount of binding between said protein molecule and an anti-protein antibody by the use of enzymatic, chromodynamic, radioactive, magnetic, or luminescent labels which are attached to either the anti-protein antibody or a secondary antibody which binds the anti-protein antibody. In addition, other high affinity ligands may be used. Immunoassays which can be used include e.g. ELISAs, Western blots and other techniques known to those of ordinary skill in the art (see Harlow and Lane, Antibodies: A Laboratory Manual, Cold Spring Harbor Laboratory Press, Cold Spring Harbor, New York, 1999 and Edwards R, Immunodiagnostics: A Practical Approach, Oxford University Press, Oxford; England, 1999). All these detection techniques may also be employed in the format of microarrays, protein-arrays, antibody microarrays, tissue microarrays, electronic biochip or protein-chip based technologies (see Schena M., Microarray Biochip Technology, Eaton Publishing, Natick, MA, 2000).

In another aspect, the invention features the use of a kit for diagnosing or prognosticating Alzheimer's disease, in a subject, or determining the or predisposition of a subject to develop Alzheimer's disease, said kit comprising: at least one reagent which is selected from the group consisting of (i) reagents that detect a transcription product of the gene coding for ADARB2 proteins (ii) reagents that detect a translation product of the gene coding for ADARB2 proteins, and/or (iii) reagents that detect a fragment of said transcription or translation products.

The kit, according to the present invention, may be particularly useful for the identification of individuals that are at risk of developing AD.

Reagents that selectively detect a transcription product and/or a translation product of the gene coding for ADARB2 proteins, preferably coding for the splice variants having SEQ ID NO: 1, or having SEQ ID NO: 2, or having SEQ ID NO: 3, or having SEQ ID NO: 4 can be sequences of various length, fragments of sequences, antibodies, aptamers, siRNA, microRNA, ribozymes. Such reagents may be used also to detect fragments, derivatives or variants thereof.

Consequently, the kit, according to the present invention, may serve as a means for targeting identified individuals for early preventive measures or therapeutic intervention prior to disease onset, before irreversible damage in the course of the disease has been inflicted. Furthermore, in preferred embodiments, the kit featured in the invention is useful for monitoring a progression of a neurodegenerative disease, in particular AD in a subject, as well as monitoring success or failure of therapeutic treatment for such a disease of said subject.

Disclosed is a method of treating or preventing a neurodegenerative disease, in particular AD, in a subject comprising the administration to said subject in need of such a treatment in a therapeutically or prophylactically effective amount and formulation an agent, agents, modulators, antagonist, agonists or antibodies which directly or indirectly affect a level, or an activity, or both said level and said activity, of (i) the gene coding for ADARB2 proteins, and/or (ii) a transcription product of the gene coding for ADARB2 proteins, and/or (iii) a translation product of the gene coding for ADARB2 proteins, and/or (iv) a fragment, or derivative, or variant of (i) to (iii). Said agent may comprise a small molecule, or it may also comprise a peptide, an oligopeptide, or a polypeptide. Said peptide, oligopeptide, or polypeptide may comprise an amino acid sequence of a translation product of the gene coding for ADARB2 proteins, or a fragment, or derivative, or a variant thereof. An agent for treating or preventing a neurodegenerative disease, in particular AD, according to the instant invention, may also consist of a nucleotide, an oligonucleotide, or a polynucleotide. Said oligonucleotide or polynucleotide may comprise a nucleotide sequence of the gene coding for ADARB2 proteins, either in sense orientation or in antisense orientation.

In preferred embodiments, the method comprises the application of per se known methods of gene therapy and/or antisense nucleic acid technology to administer said agent or agents. In general, gene therapy includes several approaches: molecular replacement of a mutated gene, addition of a new gene resulting in the synthesis of a therapeutic protein, and modulation of endogenous cellular gene expression by recombinant expression methods or by drugs. Gene-transfer techniques are described in detail (see e.g. Behr, Acc Chem Res 1993, 26: 274-278 and Mulligan, Science 1993, 260: 926-931) and include direct gene-transfer techniques such as mechanical microinjection of DNA into a cell as well as indirect techniques employing biological vectors (like recombinant viruses, especially retroviruses) or model liposomes, or techniques based on transfection with DNA co-precipitation with polycations, cell membrane pertubation by chemical (solvents, detergents, polymers, enzymes) or physical means (mechanic, osmotic, thermic, electric shocks). The postnatal gene transfer into the central nervous system has been described in detail (see e.g. Wolff, Curr Opin Neurobiol 1993, 3: 743-748).

Disclosed is a method of treating or preventing a neurodegenerative disease by means of antisense nucleic acid therapy, i.e. the down-regulation of an inappropriately expressed or defective gene by the introduction of antisense nucleic acids or derivatives thereof into certain critical cells (see e.g. Gillespie, DM&P 1992, 5: 389-395; Agrawal and Akhtar, Trends Biotechnol 1995, 13: 197-199; Crooke, Biotechnology 1992, 10: 882-6). Apart from hybridization strategies, the application of ribozymes, i.e. RNA molecules that act as enzymes, destroying RNA that carries the message of disease has also been described (see e.g. Barinaga, Science 1993, 262: 1512-1514). In preferred embodiments, the subject to be treated is a human, and therapeutic antisense nucleic acids or derivatives thereof are directed against transcription products of the gene coding for ADARB2 proteins. It is preferred that cells of the central nervous system, preferably the brain, of a subject are treated in such a way. Cell penetration can be performed by known strategies such as coupling of antisense nucleic acids and derivatives thereof to carrier particles, or the above described techniques. Strategies for administering targeted therapeutic oligodeoxynucleotides are known to those of skill in the art (see e.g. Wickstrom, Trends Biotechnol 1992, 10: 281-287). In some cases, delivery can be performed by mere topical application. Further approaches are directed to intracellular expression of antisense RNA. In this strategy, cells are transformed *ex vivo* with a recombinant gene that directs the synthesis of an RNA that is complementary to a region of target nucleic acid. Therapeutical use of intracellularly expressed antisense RNA is procedurally similar to gene therapy. A recently developed method of regulating the intracellular expression of genes by the use of double-stranded RNA, known variously as RNA interference (RNAi), can be another effective approach for nucleic acid therapy (Hannon, Nature 2002, 418: 244-251).

In further preferred embodiments, the method comprises grafting donor cells into the central nervous system, preferably the brain, of said subject, or donor cells preferably treated so as to minimize or reduce graft rejection, wherein said donor cells are genetically modified by insertion of at least one transgene encoding said agent or agents. Said transgene might be carried by a viral vector, in particular a retroviral vector. The transgene can be inserted into the donor cells by a nonviral physical transfection of DNA encoding a transgene, in particular by microinjection. Insertion of the transgene can also be performed by electroporation, chemically mediated transfection, in particular calcium phosphate transfection or liposomal mediated transfection (see Mc Celland and Pardee, Expression Genetics: Accelerated and High-Throughput Methods, Eaton Publishing, Natick, MA, 1999).

In preferred embodiments, said agent for treating and preventing AD, is a therapeutic protein which can be administered to said subject, preferably a human, by a process comprising introducing subject cells into said subject, said subject cells having been treated *in vitro* to insert a DNA segment encoding said therapeutic protein, said subject cells expressing *in vivo* in said subject a therapeutically effective amount of said therapeutic protein. Said DNA segment can be inserted into said cells *in vitro* by a viral vector, in particular a retroviral vector.

Methods of treatment or prevention, as disclosed herein , comprise the application of therapeutic cloning, transplantation, and stem cell therapy using embryonic stem cells or embryonic germ cells and neuronal adult stem cells, combined with any of the previously described cell- and gene therapeutic methods. Stem cells may be totipotent or pluripotent. They may also be organ-specific. Strategies for repairing diseased and/or damaged brain cells or tissue comprise (i) taking donor cells from an adult tissue. Nuclei of those cells are transplanted into unfertilized egg cells from which the genetic material has been removed. Embryonic stem cells are isolated from the blastocyst stage of the cells which underwent somatic cell nuclear transfer. Use of differentiation factors then leads to a directed development of the stem cells to specialized cell types, preferably neuronal cells (Lanza et al., Nature Medicine 1999, 9: 975-977), or (ii) purifying adult stem cells, isolated from the central nervous system, or from bone marrow (mesenchymal stem cells), for in vitro expansion and subsequent grafting and transplantation, or (iii) directly inducing endogenous neural stem cells to proliferate, migrate, and differentiate into functional neurons (Peterson DA, Curr. Opin. Pharmacol. 2002, 2: 34-42) Adult neural stem cells are of great potential for repairing damaged or diseased brain tissues, as the germinal centers of the adult brain are free of neuronal damage or dysfunction (Colman A, Drug Discovery World 2001, 7: 66-71).

In preferred embodiments, the subject for treatment or prevention, according to the present invention, can be a human, or a non-human experimental animal, e.g. a mouse or a rat, a domestic animal, or a non-human primate. The experimental animal can be an animal model for a neurodegenerative disorder, e.g. a transgenic mouse and/or a knock-out mouse with an AD-type neuropathology.

Disclosed is an agent, an antagonist, agonist or a modulator of an activity, or a level, or both said activity and said level, and/or of expression of at least one substance which is selected from the group consisting of (i) the gene coding for ADARB2 proteins, and/or (ii) a transcription product of the gene coding for ADARB2 proteins, and/or (iii) a translation product of .the gene coding for ADARB2 proteins, and/or (iv) a fragment, or derivative, or variant of (i) to (iii), and said agent, antagonist or agonist, or said modulator has a potential activity in the treatment of neurodegenerative diseases, in particular AD.

Further disclosed is the use of an agent, an antibody, an antagonist or agonist, or a modulator of an activity, or a level, or both said activity and said level, and/or of expression of at least one substance which is selected from the group consisting of (i) the gene coding for ADARB2 proteins, and/or (ii) a transcription product of the gene coding for ADARB2 proteins, and/or (iii) a translation product of the gene coding for ADARB2 proteins, and/or (iv) a fragment, or derivative, or variant of (i) to (iii) in the manufacture of a medicament for treating or preventing a neurodegenerative disease, in particular AD. Said antibody may be specifically immunoreactive with an immunogen which is a translation product of a gene coding for ADARB2 (preferably having SEQ ID NO: 1, or SEQ ID NO: 2, or SEQ ID NO: 3, or SEQ ID NO: 4) or a fragment, derivative or variant of such translation product..

In an additional aspect, the invention features a pharmaceutical composition comprising said agent, antibody, antagonist or agonist, or modulator and preferably a pharmaceutical carrier. Said carrier refers to a diluent, adjuvant, excipient, or vehicle with which the modulator is administered.

In one aspect, the present invention also provides a kit comprising one or more containers filled with a therapeutically or prophylactically effective amount of said pharmaceutical composition.

In a further aspect, the invention features a genetically modified non-human animal comprising a non-native ADARB2 gene sequence coding for a ADARB2 protein having SEQ ID NO: 1, or SEQ ID NO: 2, or SEQ ID NO: 3, or SEQ ID NO: 4 or a fragment thereof under the control of a transcriptional element which is not the native ADARB2 gene transcriptional control element,

wherein the expression of said gene, , and wherein the disruption or alteration of said gene sequence results in said non-human animal exhibiting a predisposition to developing signs of AD. Strategies and techniques for the generation and construction of such an animal are known to those of ordinary skill in the art (see e.g. Capecchi, Science 1989, 244: 1288-1292 and Hogan et al., Manipulating the Mouse Embryo: A Laboratory Manual, Cold Spring Harbor Laboratory Press, Cold Spring Harbor, New York, 1994. and Jackson and Abbott, Mouse Genetics and Transgenics: A Practical Approach, Oxford University Press, Oxford, England, 1999).

The invention features the use of such a genetically modified non-human animal as non-human test animal or as a control animal for screening, testing and or validating compounds, agents and modulators in the development of diagnostics and therapeutics useful for the treatment of Alzheimer's disease. The use of such a genetically modified animal in a screening method is disclosed in the instant invention.

In a further aspect the invention makes use of a cell, in which a gene sequence coding for a ADARB2 protein having SEQ ID NO: 1, or SEQ ID NO: 2, or SEQ ID NO: 3, or SEQ ID NO: 4, or a fragment thereof is expressed, or disrupted or altered for screening, testing and or validating compounds, agents and modutators in the development of diagnostics and therapeutics useful for the treatment of Alzheimer's disease. The use of such a cell in a screening method is disclosed in the instant invention..

In another aspect, the invention features a method of screening for an agent, a modulator, an antagonist or agonist for use in the treatment or prevention of AD, which agents, modulators, antagonists or agonists have an ability to alter expression or level of one or more substances selected from the group consisting of (i) the gene coding for ADARB2 protein (preferably having SEQ ID NO: 1, or SEQ ID NO: 2, or SEQ ID NO: 3, or SEQ ID NO: 4) and/or (ii) a transcription product of the gene coding for ADARB2 protein (preferably having SEQ ID NO: 1, or SEQ ID NO: 2, or SEQ ID NO: 3, or SEQ ID NO: 4), and/or (iii) a translation product of the gene coding for ADARB2 protein (preferably having SEQ ID NO: 1, or SEQ ID NO: 2, or SEQ ID NO: 3. or SEQ ID NO: 4), and/or (iv) a fragment of (i) to (iii). This screening method comprises (a) contacting a cell with a test compound, and (b) measuring the level, and/or the expression of one or more substances recited in (i) to (iv), and (c) measuring the level and/or the expression of said substances in a control cell not contacted with said test compound, and (d) comparing the levels and/or the expression of the substance in the cells of step (b) and (c), wherein an alteration in the level and/or expression of said substances in the contacted cells indicates that the test compound is an agent, modulator, antagonist or agonist for use in the treatment of Alzheimer's disease

In one further aspect, the invention features a method of screening for an agent, a modulator, an antagonist or agonist for use in the treatment of AD, which agents, modulators antagonists or agonists have an ability to alter expression or level of one or more substances selected from the group consisting of (i) the gene coding for ADARB2 protein (preferably having SEQ ID NO: 1, or SEQ ID NO: 2, or SEQ ID NO: 3, or SEQ ID NO: 4), and/or (ii) a transcription product of the gene coding for ADARB2 protein (preferably having SEQ ID NO: 1, or SEQ ID NO: 2, or SEQ ID NO: 3, or SEQ ID NO: 4), and/or (iii) a translation product of the gene coding for ADARB2 protein (preferably having SEQ ID NO: 1, or SEQ ID NO: 2, or SEQ ID NO: 3, or SEQ ID NO: 4), and/or (iv) a fragment of (i) to (iii), comprising (a) administering a test compound to a non-human test animal which is predisposed to developing or has already developed signs and symptoms of Alzheimer's disease, and (b) measuring the level and/or expression of one or more substances recited in (i) to (iv), and (c) measuring the level and/or expression of said substances in a non-human control animal which is predisposed to developing or has already developed said signs and symptoms of Alzheimer's disease , and to which non-human animal no such test compound has been administered, and (d) comparing the level and/or expression of the substances in the animals of step (b) and (c), wherein an alteration in the level and/or expression of substances in the non-human test animal indicates that the test compound is an agent, modulator, antagonist or agonist for use in the treatment of Alzheimer's diseases.

In another embodiment, the present invention provides a method for producing a medicament comprising the steps of (i) identifying an agent, modulator, antagonists or agonists of neurodegenerative diseases by a method of the aforementioned screening assays and (ii) admixing said agent, modulator, antagonist or agonist with a pharmaceutical carrier. However, said agent, modulator, antagonist or agonist may also be identifiable by other types of screening methods and assays.

Disclosed is an assay for testing a compound or compounds, preferably for screening a plurality of compounds in high-throughput format, to determine the degree of inhibition of binding or the enhancement of binding between a ligand and ADARB2 protein (preferably having SEQ ID NO: 1, or SEQ ID NO: 2, or SEQ ID NO: 3, or SEQ ID NO: 4), or a fragment, or derivative, or variant thereof and/or to determine the degree of binding of said compounds to ADARB2 protein (preferably having SEQ ID NO: 1, or SEQ ID NO: 2, or SEQ ID NO: 3, or SEQ ID NO: 4), or a fragment, or derivative, or variant thereof. For determination of inhibition of binding between a ligand and ADARB2 protein, or a fragment, or derivative, or variant thereof, said screening assay comprises the steps of (i) adding a liquid suspension of said ADARB2 protein, or a fragment, or derivative, or variant thereof, to a plurality of containers, and (ii) adding a compound or a plurality of compounds to be screened for said inhibition to said plurality of containers, and (iii) adding a detectable, preferably a fluorescently labelled ligand to said containers, and (iv) incubating said ADARB2 protein, or said fragment, or derivative or variant thereof, and said compound or plurality of compounds, and said detectable, preferably fluorescently labelled ligand, and (v) measuring the amounts of ligand, preferably its fluorescence associated with said ADARB2 protein, or with said fragment, or derivative, or variant thereof, and (vi) determining the degree of inhibition by one or more of said compounds of binding of said ligand to said ADARB2 protein, or said fragment, or derivative, or variant thereof. It might be preferred to reconstitute said ADARB2 translation product, or fragment, or derivative, or variant thereof into artificial liposomes to generate the corresponding proteoliposomes to determine the inhibition of binding between a ligand and said ADARB2 translation product. Methods of reconstitution of ADARB2 translation products from detergent into liposomes have been detailed (Schwarz et al., Biochemistry 1999, 38: 9456-9464; Krivosheev and Usanov, Biochemistry-Moscow 1997, 62: 1064-1073). Instead of utilizing a fluorescently, labelled ligand, it might in some aspects be preferred to use any other detectable label known to the person skilled in the art, e.g. radioactive labels, and detect it accordingly. Said method may be useful for the identification of novel compounds as well as for evaluating compounds which have been improved or otherwise optimized in their ability to inhibit the binding of a ligand to a gene product of the gene coding for ADARB2 protein, or a fragment, or derivative, or variant thereof. One example of a fluorescent binding assay, in this case based on the use of carrier particles, is disclosed and described in patent application WO00/52451. A further example is the competitive assay method as described in patent WO02/0122 Preferred signal detection methods for screening assays of the instant invention are described in the following patent applications: WO96/13744, WO98/16814, WO98/23942, WO99/17086, WO99/34195, WO00/66985, WO01/59436, and WO01/59416.

In one further embodiment, the present invention provides a method for producing a medicament comprising the steps of (i) identifying a compound as an inhibitor of binding between a ligand and a gene product of the gene coding for ADARB2 proteins by the aforementioned inhibitory binding assay and (ii) admixing the compound with a pharmaceutical carrier. However, said compound mary also be identifiable by other types of screening assays.

Further disclosed is an assay for testing a compound or compounds, preferably for screening a plurality of compounds in high-throughput format to determine the degree of binding of said compounds to ADARB2 protein (preferably having SEQ ID NO: 1, or SEQ ID NO: 2, or SEQ ID NO: 3, or SEQ ID NO: 4), or to a fragment, or derivative, or variant thereof, said screening assay comprises (i) adding a liquid suspension of said ADARB2 protein, or a fragment, or derivative, or variant thereof, to a plurality of containers, and (ii) adding a detectable, preferably a fluorescently labelled compound or a plurality of detectable, preferably fluorescently labelled compounds to be screened for said binding to said plurality of containers, and (iii) incubating said ADARB2 protein, or said fragment, or derivative, or variant thereof, and said detectable, preferably fluorescently labelled compound or detectable, preferably fluorescently labelled compounds, and (iv) measuring the amounts of compound, preferably its fluorescence, associated with said ADARB2 protein, or with said fragment, or derivative, or variant thereof, and (v) determining the degree of binding by one or more of said compounds to said ADARB2 protein, or said fragment, or derivative, or variant thereof. In this type of assay it might be preferred to use a fluorescent label. However, any other type of detectable label might also be employed. Also in this type of assay it might be preferred to reconstitute an ADARB2 translation product or a fragment, or derivative, or variant thereof into artificial liposomes as described in the present invention. Said assay methods may be useful for the identification of novel compounds as well as for evaluating compounds which have been improved or otherwise optimized in their ability to bind to ADARB2 protein, or a fragment, or derivative, or variant thereof.

In one further embodiment, the present invention provides a method for producing a medicament comprising the steps of (i) identifying a compound as a binder to a gene product of the gene coding for ADARB2 proteins by the aforementioned binding assays and (ii) admixing the compound with a pharmaceutical carrier. However, said compound may also be identifiable by other types of screening assays.

In another embodiment, the present invention provides for a medicament obtainable by any of the methods according to the herein claimed screening assays. In one further embodiment, the instant invention provides for a medicament obtained by any of the methods according to the herein claimed screening assays.

In general, the aforementioned assay and screening methods as well as potential drug molecules (e.g. agents modulators, antagonists, agonists) identified therefrom have applicability in relation to the treatment or prevention of neurodegenerative diseases, in particular Alzheimer's disease.

Another aspect of the present invention features protein molecules being translation products of the gene coding for ADARB2 and the use of said protein molecules (preferably having SEQ ID NO: 1, or SEQ ID NO: 2, or SEQ ID NO: 3, or SEQ ID NO: 4), or fragments thereof, as diagnostic targets for detecting Alzheimer's disease.

The present invention further features protein molecules being translation products of the gene coding for ADARB2 and the use of said protein molecules (preferably having SEQ ID NO: 1, or SEQ ID NO: 2, or SEQ ID NO: 3, or SEQ ID NO: 4), or fragments thereof, as screening targets for modulators, agents or compounds preventing, or treating, or ameliorating Alzheimer's disease.

The present invention features the use of antibodies which are specifically immunoreactive with an immunogen, wherein said immunogen is a translation product of the ADARB2 gene coding for ADARB2 proteins (preferably having SEQ ID NO: 1, or SEQ ID NO: 2, or SEQ ID NO: 3, or SEQ ID NO: 4), or fragments thereof, for detecting the pathological state of a cell in a sample obtained from a subject, comprising immunocytochemical staining of said cell with said antibody, wherein an altered degree of staining, or an altered staining pattern in said cell compared to a cell representing a known health status indicates a pathological state of said cell which relates to AD. The immunogen may comprise immunogenic or antigenic epitopes or portions of a translation product of said gene, wherein said immunogenic or antigenic portion of a translation product is a polypeptide, and wherein said polypeptide elicits an antibody response in an animal, and wherein said polypeptide is immunospecifically bound by said antibody. Methods for generating antibodies are well known in the art (see Harlow et al., Antibodies, A Laboratory Manual, Cold Spring Harbor Laboratory Press, Cold Spring Harbor, New York, 1988). The term "antibody", as employed in the present invention, encompasses all forms of antibodies known in the art, such as polyclonal, monoclonal, chimeric, recombinatorial, anti-idiotypic, humanized, or single chain antibodies, as well as fragments thereof (see Dubel and Breitling, Recombinant Antibodies, Wiley-Liss, New York, NY, 1999). Antibodies of the present invention are useful, for instance, in a variety of diagnostic and therapeutic methods, based on state-in-the-art techniques (see Harlow and Lane, Using Antibodies: A Laboratory Manual, Cold Spring Harbor Laboratory Press, Cold Spring Harbor, New York, 1999 and Edwards R., Immunodiagnostics: A Practical Approach, Oxford University Press, Oxford, England, 1999) such as enzyme-immunoassays (e.g. enzyme-linked immunosorbent assay, ELISA), radioimmunoassays, chemoluminescence-immuno assays, Western-blot, immunoprecipitation and antibody microarrays. These methods involve the detection of translation products of the ADARB2 gene, or fragments thereof.

Immunocytochemical staining of a cell can be carried out by a number of different experimental methods well known in the art. It might be preferred, however, to apply an automated method for the detection of antibody binding, wherein the determination of the degree of staining of a cell, or the determination of the cellular or subcellular staining pattern of a cell, or the topological distribution of an antigen on the cell surface or among organelles and other subcellular structures within the cell, are carried out according to the method described in US6150173.

Other features and advantages of the invention will be apparent from the following description of figures and examples which are illustrative only and not intended to limit the remainder of the disclosure in any way.

FIGURES:

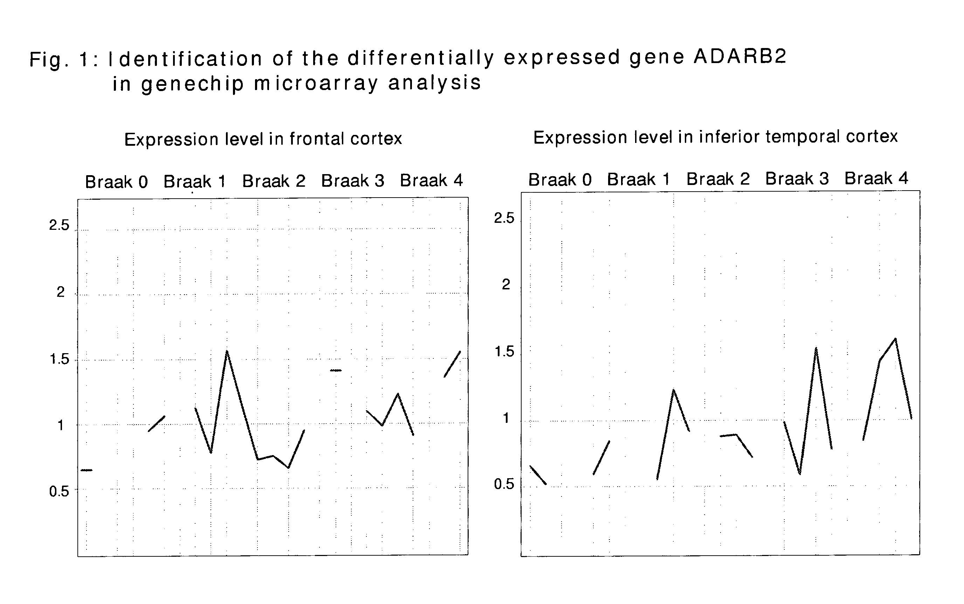

Figure 1 shows the identification of differences in the levels of ADARB2 gene derived mRNA in human brain tissue samples from individuals corresponding to different Braak stages as measured and compared by GeneChip analyses. It indicates that the levels of the respective mRNA species correlate quantitatively with AD progression and thus are indicative for AD as measured by the neuropathological staging of brain tissue samples according to Braak and Braak (Braak staging). cRNA probes of frontal cortex as well as of inferior temporal cortex each of 5 different donors with Braak stage 0 (C011, C012, C026, C027, and C032), 7 different donors with Braak stage 1 (C014, C028, C029, C030, C036, C038, and C039), 5 different donors with Braak stage 2 (C008, C031, C033, C034, and DE03), 4 different donors with Braak stage 3 (C025, DE07, DE11, and C057), and 4 different donors with Braak stage 4 (P012, P046, P047, and P068) have been applied to an analysis of an Affymetrix Human Genome U133 Plus 2.0 Array respectively. Differences reflecting an up-regulation of the ADARB2 gene progressively with Braak stages predominantly in inferior temporal tissue are shown.

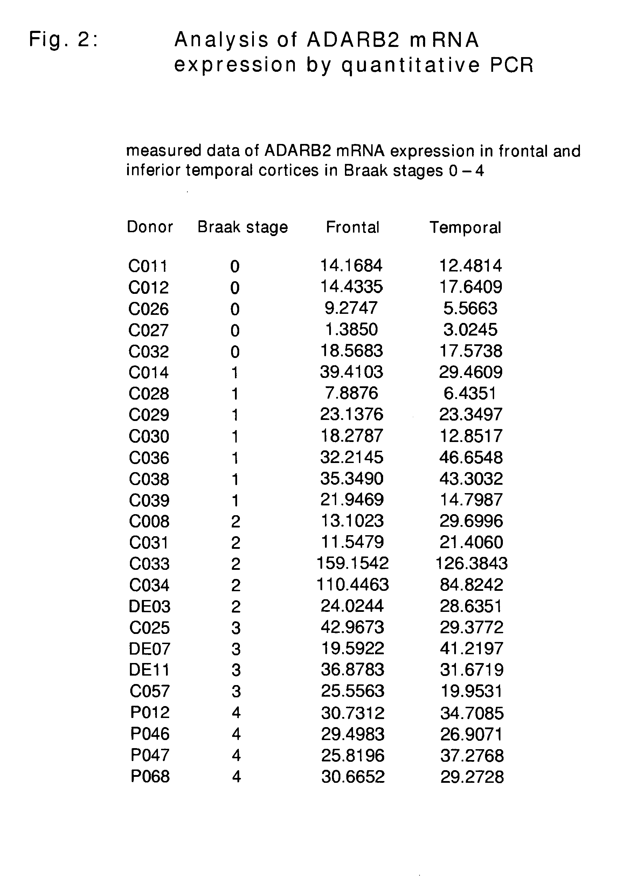

Figure 2 lists the data for the verification of differences in the levels of ADARB2 gene derived mRNA in human brain tissue samples from individuals corresponding to different Braak stages indicative for AD as measured by quantitative RT-PCR analysis. Quantitative RT-PCR using the Roche Lightcycler rapid thermal cycling technique was performed applying cDNA of the frontal cortex (Frontal) and the inferior temporal cortex (Temporal) of the same donors as used for GeneChip analysis. The data were normalized to values of cyclophilin B a standard gene that showed no significant differences in its gene expression levels. The comparison between samples of the lowest Braak stage 0 with samples representing high Braak stage 4 clearly demonstrates a substantial difference in gene expression level of ADARB2.

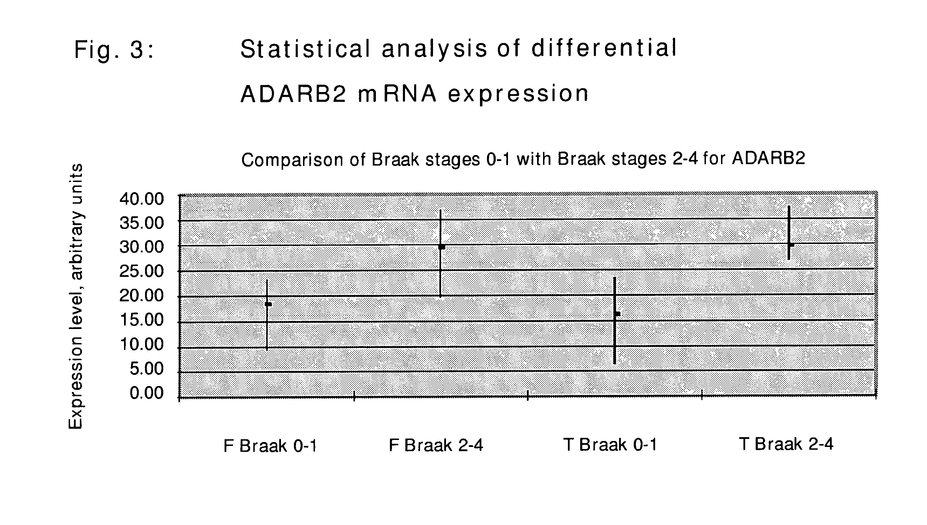

Figure 3 shows the analysis of absolute levels of ADARB2 gene derived mRNA in human brain tissue samples from individuals corresponding to different Braak stages indicative for AD as measured by quantitative RT-PCR and using statistical method of the median at 98%-confidence level (Sachs L (1988) Statistische Methoden: Planung und Auswertung. Heidelberg New York, p. 60). The data were calculated by defining control groups including subjects with Braak stages 0 to 1, which are compared with the data calculated for the defined groups with advanced AD pathology including Braak stages 2 to 4. A substantial difference reflecting an up-regulation of ADARB2 is shown in frontal as well as in inferior temporal cortices corroborating results from the GeneChip analysis. A significant difference reflecting an up-regulation of ADARB2 is shown comparing inferior temporal cortex (T) of Braak stage 0-1 with Braak stage 2-4.

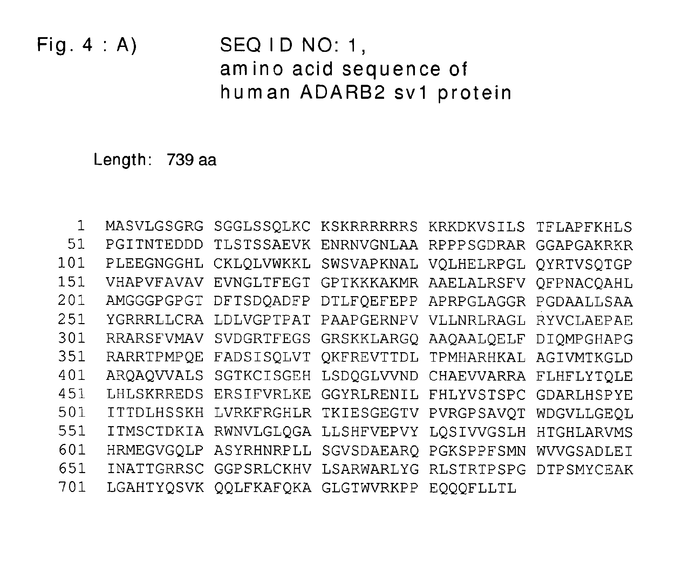

Figure 4A discloses SEQ ID NO: 1, the amino acid sequence of the human ADARB2 protein (splice variant 1, sv1) (UniProt primary accession number Q9NS39). This ADARB2 protein comprises 739 amino acids.

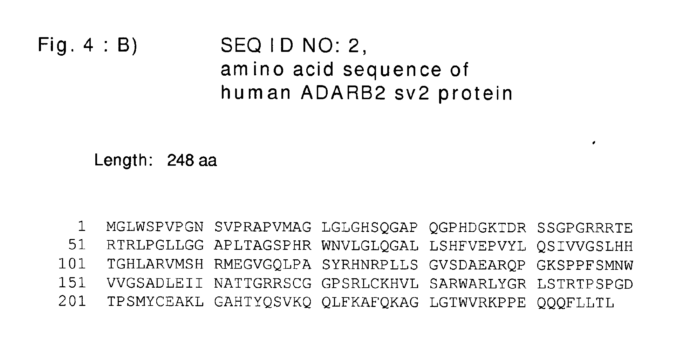

Figure 4B discloses SEQ ID NO: 2, the amino acid sequence of the human ADARB2 protein (splice variant 2, sv2) (UniProt primary accession number Q5VW42). This ADARB2 protein comprises 248 amino acids.

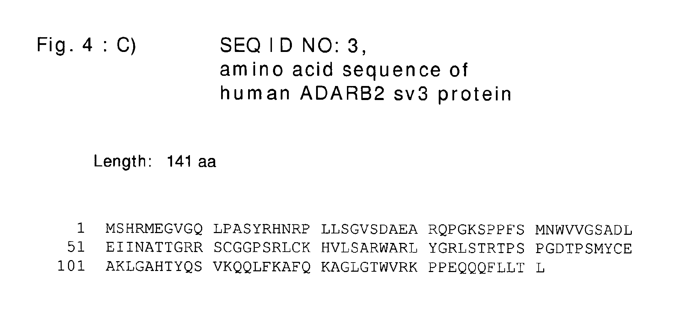

Figure 4C discloses SEQ ID NO: 3, the amino acid sequence of the human ADARB2 protein (splice variant 3, sv3) (UniProt primary accession number Q5VW43). This ADARB2 protein comprises 141 amino acids.

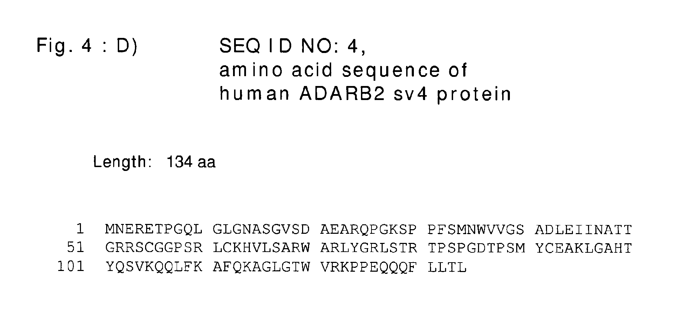

Figure 4D discloses SEQ ID NO: 4, the amino acid sequence of the human ADARB2 protein (splice variant 4, sv4) (UniProt primary accession number Q86X17). This ADARB2 protein comprises 134 amino acids.

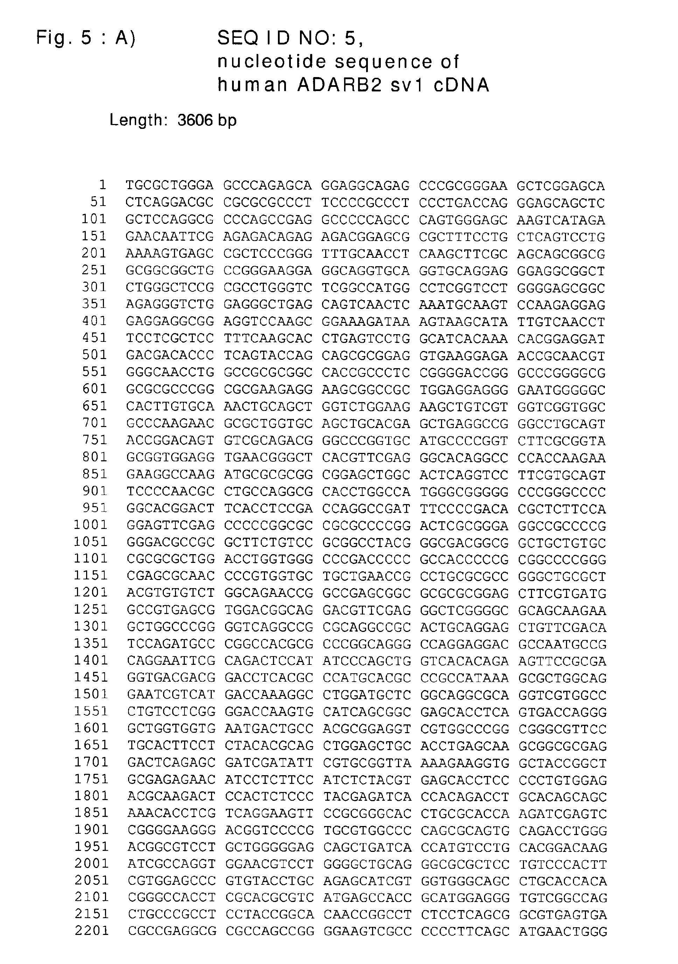

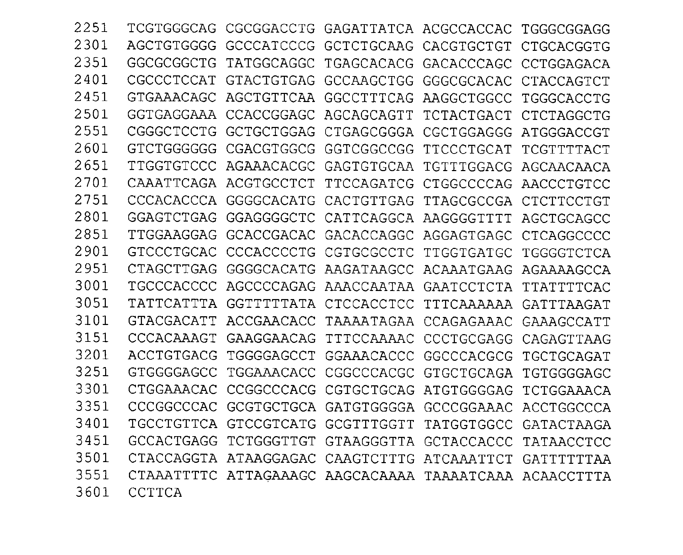

Figure 5A shows SEQ ID NO: 5, the nucleotide sequence of the human ADARB2 cDNA (splice variant 1, sv1) (Ensembl transcript ID number ENST00000381312) encoding the ADARB2 sv1 protein, comprising 3606 nucleotides.

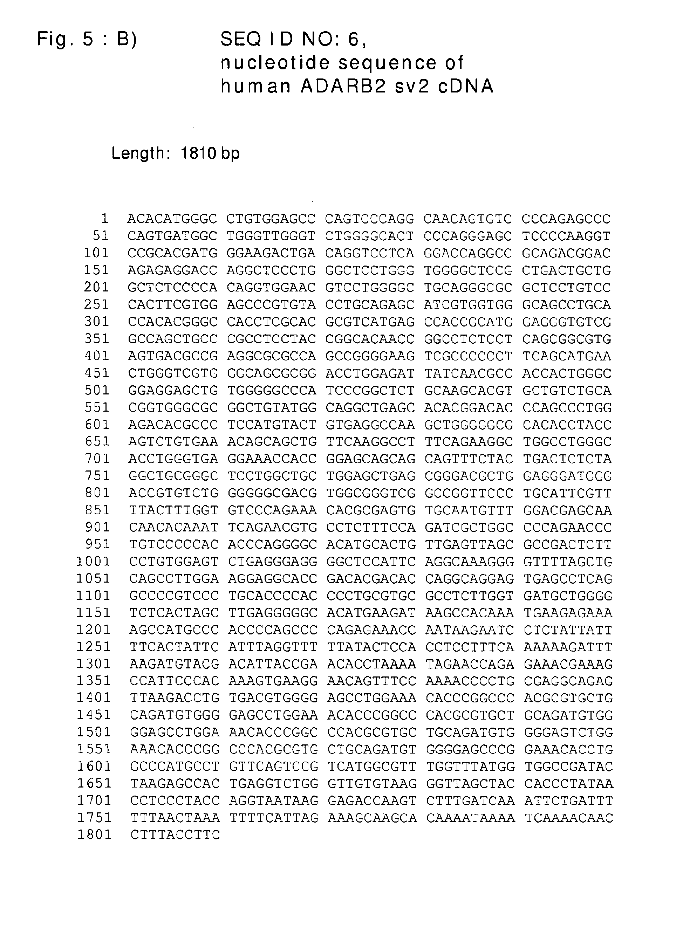

Figure 5B shows SEQ ID NO: 6, the nucleotide sequence of the human ADARB2 cDNA (splice variant 2, sv2) (Ensembl transcript ID number ENST00000381310) encoding the ADARB2 sv2 protein, comprising 1810 nucleotides.

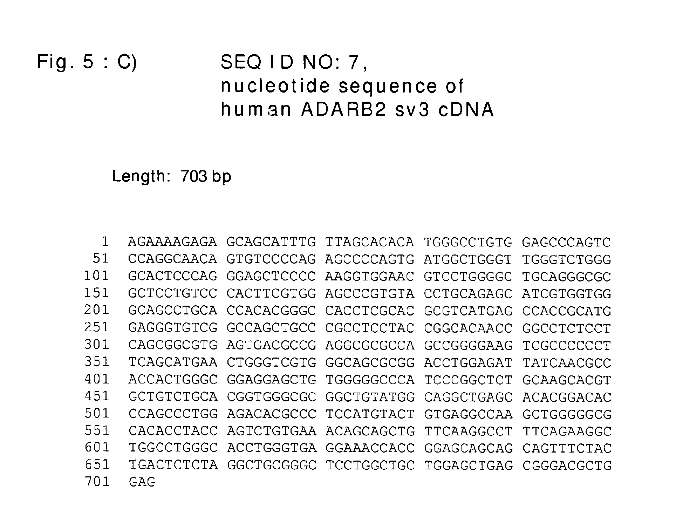

Figure 5C shows SEQ ID NO: 7, the nucleotide sequence of the human ADARB2 cDNA (splice variant 3, sv3) (Ensembl transcript ID number ENST00000381305) encoding the ADARB2 sv3 protein, comprising 703 nucleotides.

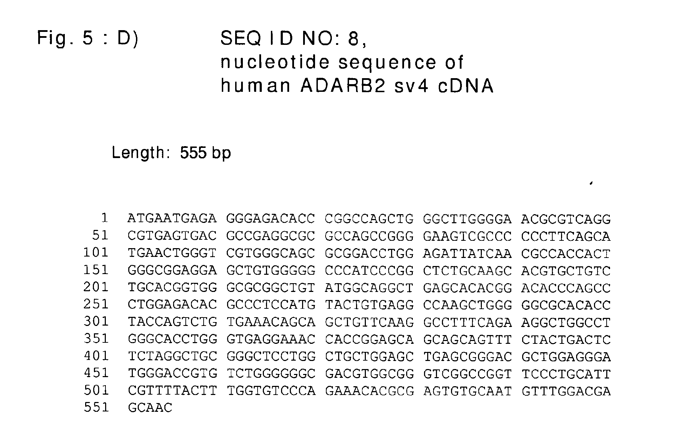

Figure 5D shows SEQ ID NO: 8, the nucleotide sequence of the human ADARB2 cDNA (splice variant 4, sv4) (Ensembl transcript ID number ENST00000337857) encoding the ADARB2 sv4 protein, comprising 555 nucleotides.

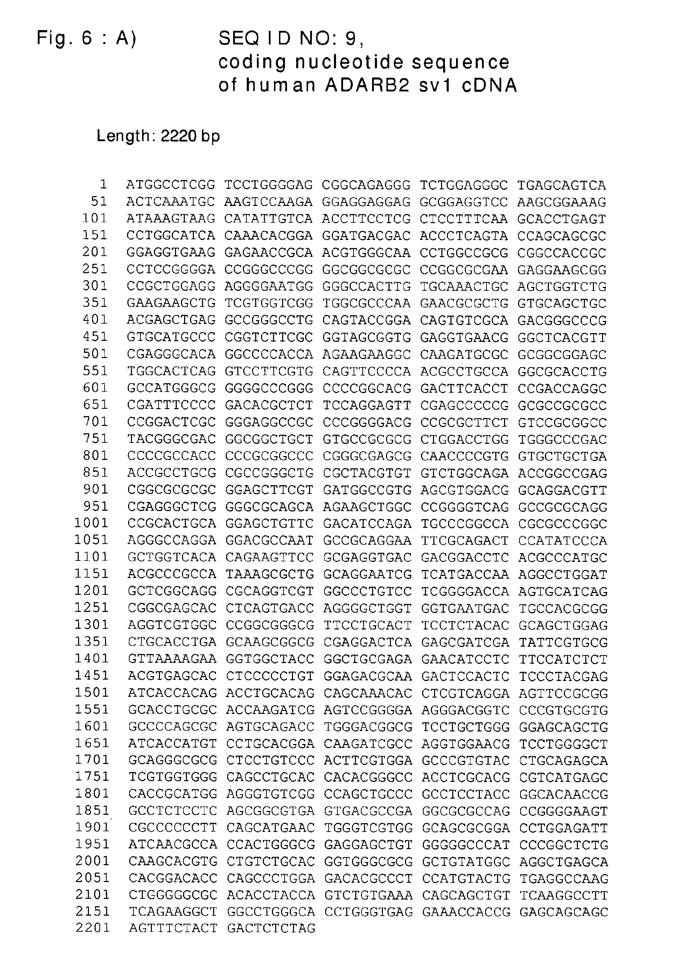

Figure 6A depicts SEQ ID NO: 9, the coding sequence (cds) of the human ADARB2 sv1, comprising 2220 nucleotides, harbouring nucleotides 327 to 2546 of SEQ ID NO. 5.

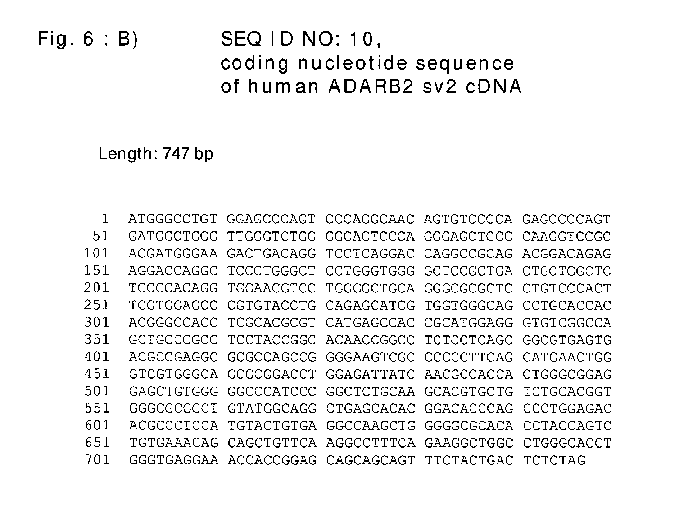

Figure 6B depicts SEQ ID NO: 10, the coding sequence (cds) of the human ADARB2 sv2, comprising 747 nucleotides, harbouring nucleotides 5 to 751 of SEQ ID NO. 6.

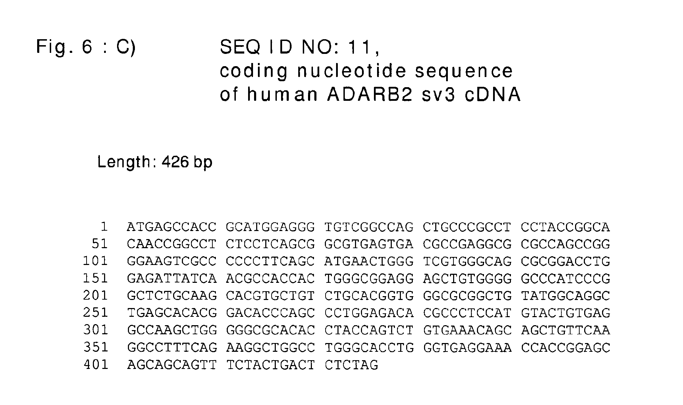

Figure 6C depicts SEQ ID NO: 11, the coding sequence (cds) of the human ADARB2 sv3, comprising 426 nucleotides, harbouring nucleotides 236 to 661 of SEQ ID NO. 7.

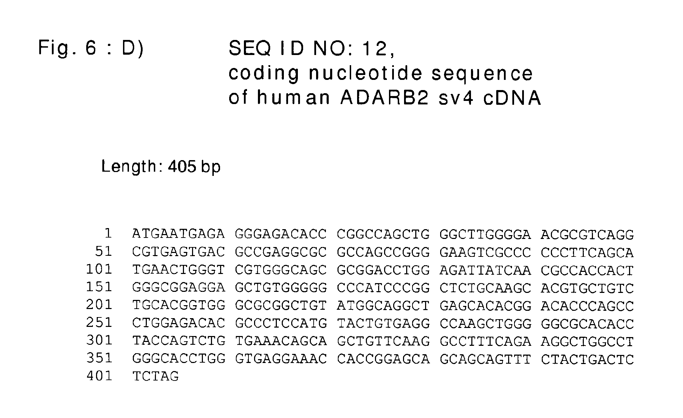

Figure 6D depicts SEQ ID NO: 12, the coding sequence (cds) of the human ADARB2 sv4, comprising 405 nucleotides, harbouring nucleotides 1 to 405 of SEQ ID NO. 8.

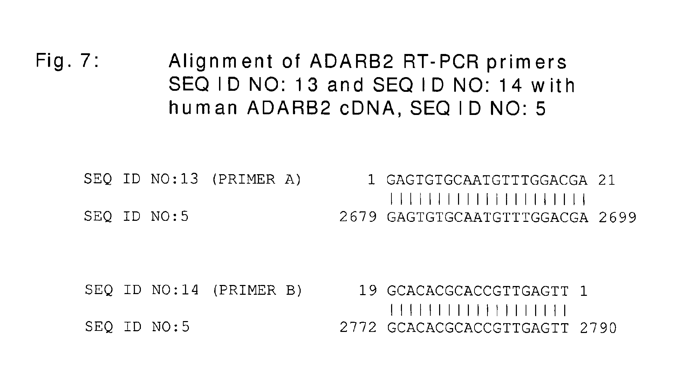

Figure 7 depicts the sequence alignment of the primers used for ADARB2 transcription level profiling (primer A, SEQ ID NO: 13 and primer B, SEQ ID NO: 14) by quantitative RT-PCR with the corresponding clippings of SEQ ID NO: 5, ADARB2 cDNA.

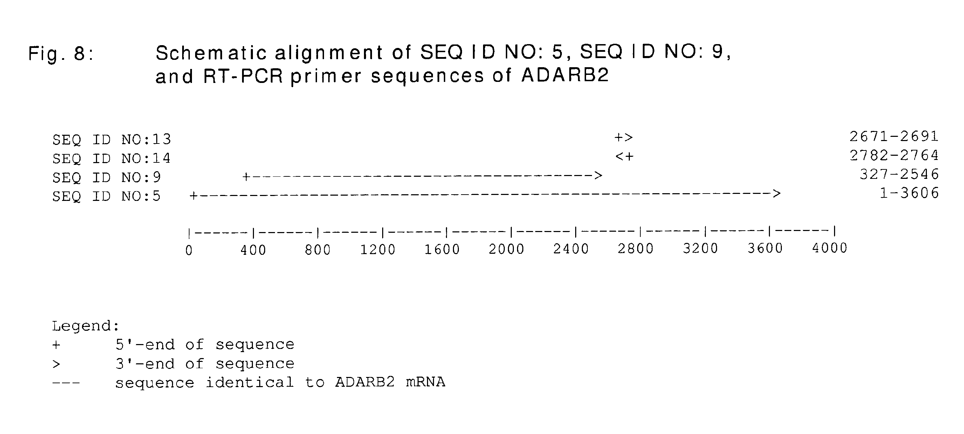

Figure 8 schematically charts the alignment of the ADARB2 cDNA sequence SEQ ID NO: 5, the ADARB2 coding sequence SEQ ID NO: 9 and both primer sequences used for ADARB2 transcription level profiling (SEQ ID NO: 13, SEQ ID NO: 14). Sequence positions are indicated on the right side.

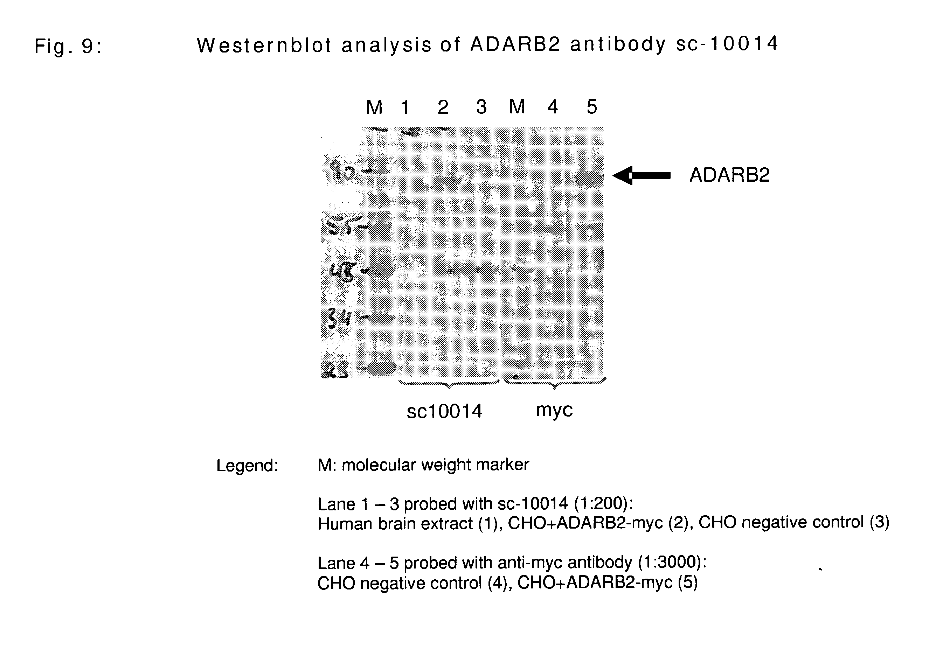

Figure 9 shows an immunoblot (Western blot) analysis of the affinity-purified polyclonal goat anti-ADARB2 antiserum sc-10014 (Santa Cruz). Protein extracts from human brain tissue homogenates and from lysates of either stably transfected CHO cells overexpressing C-terminally myc-tagged human ADARB2 protein or of naïve CHO cells (negative controls) were subjected to SDS-PAGE, blotted onto polyvinylidene difluoride membrane, and probed with either sc-10014 or anti-myc antibody, followed by an appropriate horseradish-peroxidase conjugate secondary antiserum. Blots were soaked with enhanced chemiluminescence substrate, and luminescence was detected on X-ray films. Lane 1 contains human brain neocortex protein extract, lanes 2 and 5 contain protein extract from myc-tagged human ADARB2 overexpressing transfected CHO cells, and lanes 3 and 4 contain protein extract from naïve CHO cells. Lanes 1 to 3 were probed with sc-10014 (1:200); lanes 4 and 5 were probed with an anti-myc antibody (1:3000). Lanes marked with "M" contain molecular weight marker.

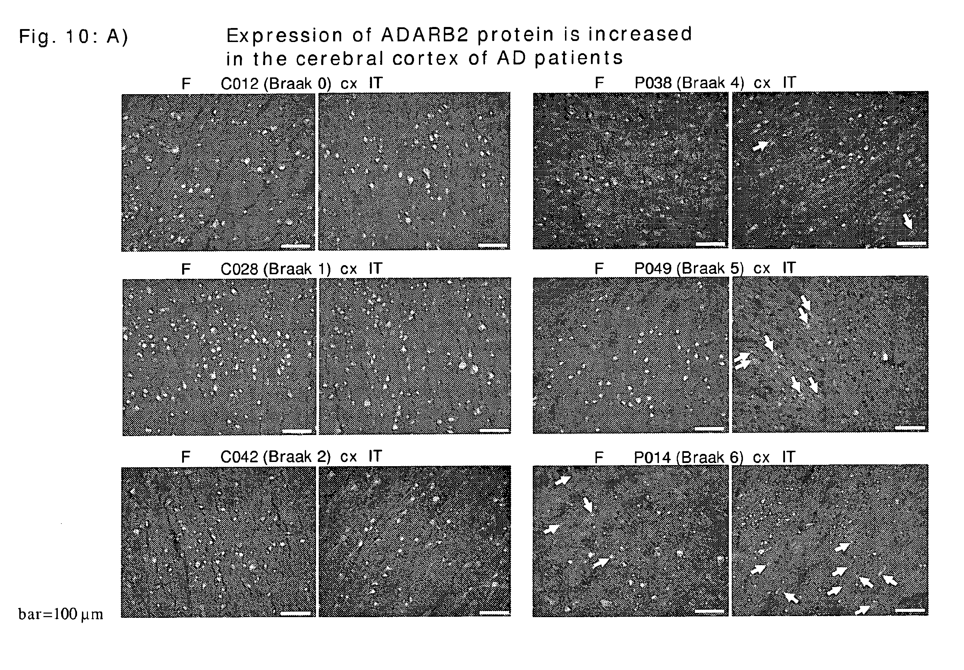

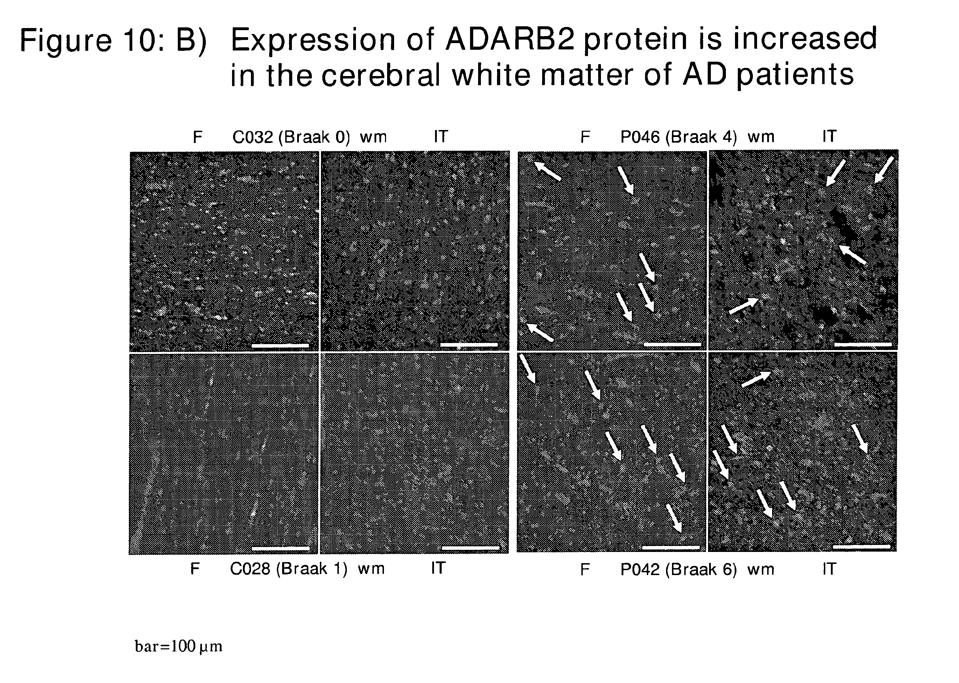

Figures 10A and 10B exemplifies an increased ADARB2 protein expression in cerebral cortex as well as in cerebral white matter observed in human brain specimens from AD patient in comparison to brain specimens from age-matched non AD control individuals. Depicted are double-immunofluorescence micrographs of acetone-fixed cryostat sections of fresh-frozen post-mortem human forebrain specimens from AD patients and age-matched non AD control donors at the Braak stages indicated in parentheses. Specific ADARB2 immunoreactivity is revealed by the affinity-purified polyclonal rabbit anti- ADARB2 antiserum sc-10014 followed by AlexaFluor-488 conjugated goat anti-rabbit IgG secondary antiserum (Molecular Probes/invitrogen), visualized as green signals. The neuron-specific marker protein NeuN is detected by the mouse monoclonal anti-NeuN antibody (Chemicon) followed by Cy3-conjugated goat anti-mouse IgG secondary antiserum (Jackson/Dianova), visualized in red colour. Nuclei are stained blue by DAPI (Sigma). Scale bars represent 100 μm in length. Abbreviations indicate: F frontal, IT inferior temporal, cx telencephalic neocortex, wm telencephalic white matter. The findings presented here are representative and show a substantial difference between control and AD samples with respect to ADARB2 expression at the protein level. In the controls, only very few - if any - astrocytes (cell bodies and processes) with slightly to moderately elevated ADARB2 immunoreactivity levels may be distinguished from the cortical neuropil (figure 10 A) or in the white matter (figure 10 B). In contrast to the controls, the AD patients' specimens consistently show an elevated frequency of clearly ADARB2 immunopositive, probably reactive astrocytes (cell bodies and processes), both in the cortex (figure 10 A) and in the white matter (figure 10 B). This finding is in agreement with the qPCR profiling data showing

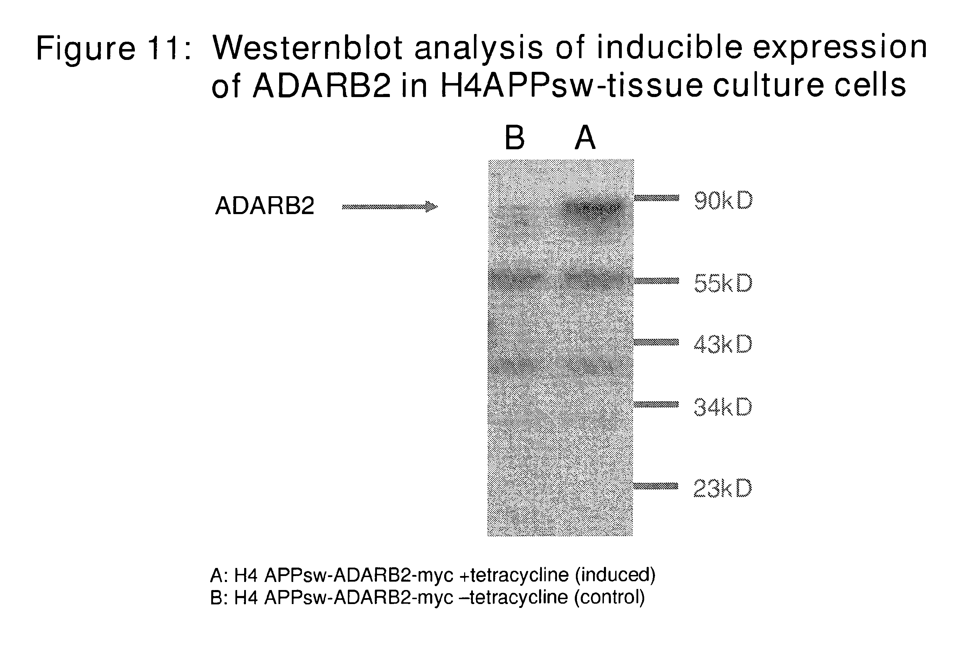

Figure 11 shows Western blot analysis of inducible ADARB2 protein expression in H4-neuroglioma cells stably co-expressing the Swedish Mutant APP. ADARB2 was myc-tagged at the C-terminus and introduced into tissue culture cells. Expression of ADARB2 is under the control of the tet-operator-sequence fused to the CMV-promoter. Upon addition of 1 μg/ml tetracycline into the medium the expression of ADARB2 is switched on. Cells were harvested, lysed and subjected to Western Blot analysis using an antibody directed against the myc-epitope at a 1:3000 dilution. The arrow points to a strong band running at approx. 85 kDa. In the absence of tetracycline no corresponding band running at the same molecular weight is visible.

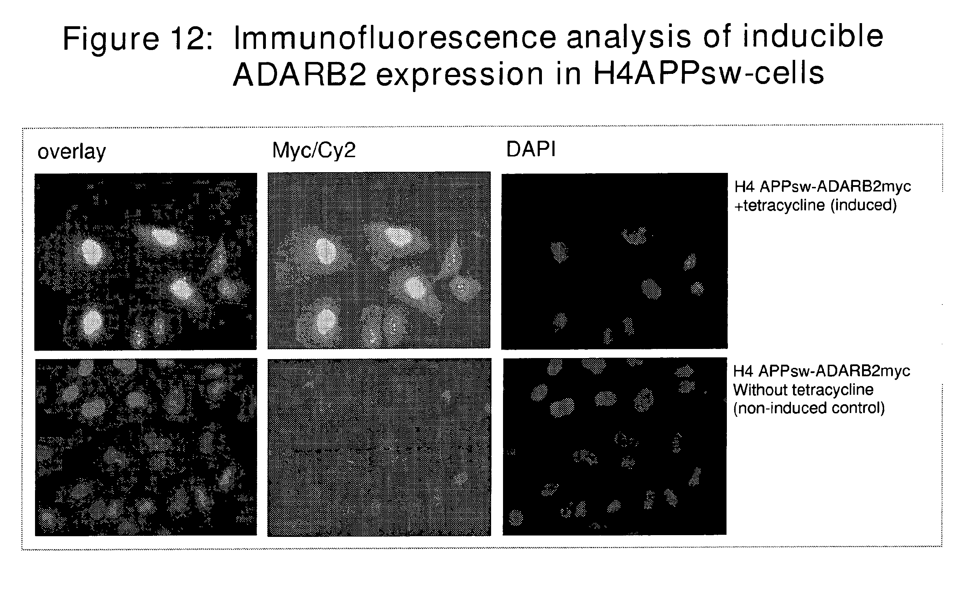

Figure 12 shows the immunofluorescence analysis of inducible ADARB2 protein expression in H4-neuroglioma cells stably co-expressing the Swedish Mutant APP and the tet-repressor. ADARB2 was myc-tagged at the C-terminus and introduced into tissue culture cells. Expression of ADARB2 is under the control of the tet-operator-sequence fused to the CMV-promoter. Upon addition of 1 μg/ml tetracycline into the medium the expression of ADARB2 in the cells that were seeded onto a glass cover slip is switched on. After 24 hours of incubation, cells where fixed with methanol for immunofluorescence analysis and expression of ADARB2 was detected using an antibody directed against the myc-epitope at a 1:3000 dilution followed by incubation with a fluorescently labelled antibody directed against the anti-myc antibody (1:1000). Cells were then mounted onto a microscope slide and analysed under a fluorescence microscope. ADARB2 is expressed in the nucleus of the cells visible by the strong green fluorescence in the upper left and middle pictures. In cells were expression is comparably lower green speckles in the nucleus of the cells become visible most probably constituting nucleoli where RNA-processing and ribosome biogenesis has been reported to take place (e.g. two cells at the most right in the upper panel). The blue colour in the upper and lower left and right pictures are indicative of the nucleus of the cells and have been visualized by means of DAPI (1:1000). The clear overlap of the blue and green fluorescence clearly indicates the expression of ADARB2 in the nucleus of the cells. In the lower panel control cells have been analysed in parallel and no green fluorescence can be detected.

EXAMPLES: Identification and verification of Alzheimer's disease related differentially expressed genes in human brain tissue samples.

In order to identify specific differences in the expression of genes that are associated with AD, GeneChip microarray (Affymetrix) analyses were performed with a diversity of cRNA probes derived from human brain tissue specimens from clinically and neuropathologically well characterized individuals. This technique is widely used to generate expression profiles of multiple genes and to compare populations of mRNA present in different tissue samples. In the present invention, mRNA populations present in selected post-mortem brain tissue specimens (frontal and inferior temporal cortex) were analyzed. Tissue samples were derived from individuals that could be grouped into different Braak stages reflecting the full range between healthy control individuals (Braak 0) and individuals that suffered from AD signs and symptoms (Braak 4). Verification of the differential expression of individual genes was performed applying real-time quantitative PCR using gene-specific oligonucleotides. Furthermore specific differences between healthy and disease stages were analysed at the protein level using gene product specific antibodies for immunohistochemical analyses. The methods were designed to specifically detect differences of expression levels at early Braak stages, which is indicative for pathological events occurring early in the course of the disease. Thus, said genes identified to be differential are effectively implicated in the pathogenesis of AD.

(i) Brain tissue dissection from patients with AD:

Brain tissues from AD patients and age-matched control subjects, were collected. Within 6 hours post-mortem time the samples were immediately frozen on dry ice. Sample sections from each tissue were fixed in paraformaldehyde and neuropathologically staged at various stages of neurofibrillary pathology according to Braak and Braak into Braak stages (0-6). Brain areas for differential expression analysis were identified and stored at -80 °C until RNA extractions were performed.

(ii) Isolation of total mRNA:

Total RNA was extracted from frozen post-mortem brain tissue by using the RNeasy kit (Qiagen) according to the manufacturer's protocol. The accurate RNA concentration and the RNA quality were determined applying the Eukaryote total RNA Nano LabChip system by using the 2100 Bioanalyzer (Agilent Technologies). For additional quality testing of the prepared RNA, i.e. exclusion of partial degradation and testing for DNA contamination, specifically designed intronic GAPDH oligonucleotides and genomic DNA as reference control were utilised to generate a melting curve with the LightCycler technology (Roche) as described in the supplied protocol by the manufacturer.

(iii) Probe synthesis:

Here, total RNA was used as starting material, which had been extracted as described above (ii). For production of cDNAs, the cDNA Synthesis System was performed according to the manufacturer's protocol (Roche). cDNA samples were transcribed to cRNA and labeled with biotin applying the in vitro-transcription T7-Megascript-Kit (Ambion) according to the manufacturer's protocol. The cRNA quality was determined applying the mRNA Smear Nano LabChip system using the 2100 Bioanalyzer (Agilent Technologies). The accurate cRNA concentration was determined by photometric analysis (OD260/280 nm).

(iv) GeneChip hybridization:

The purified and fragmented biotin labeled cRNA probes together with commercial spike controls (Affymetrix) bioB (1.5 pM), bioC (5 pM), bioD (25 pM), and cre (100 pM) were resuspended each at a concentration of 60 ng/μl in hybridization buffer (0.1 mg/ml Herring Sperm DNA, 0.5 mg/ml Acetylated BSA, 1 x MES) and subsequently denaturated for 5 min at 99 °C. Subsequently, probes were applied each onto one prehybridized (1 x MES) Human Genome U133 Plus 2.0 Array (Affymetrix). Array hybridization was performed over night at 45°C and 60 rpm. Washing and staining of the microarrays followed according to the instruction EukGe_WS2v4 (Affymetrix) controlled by GeneChip Operating System (GCOS) 1.2 (Affymetrix).

(v) GeneChip data analysis:

Fluorescence raw data were taken using the GeneScanner 3000 (Affymetrix) controlled by GCOS 1.2 software (Affymetrix). Data analysis was performed using DecisionSite 8.0 for Functional Genomics (Spotfire): raw data were delimitated to those that were flagged as "present" by the GCOS 1.2 software (Affymetrix); normalization of raw data was performed by percentile value; detection of differential mRNA expression profiles was performed using profile search tool of the Spotfire software. The result of such GeneChip data analysis for the gene coding for ADARB2 protein is shown in Figure 1.

(vi) Quantitative RT-PCR:

Positive corroboration of differential ADARB2 gene expression was performed using the LightCycler technology (Roche). This technique features rapid thermal cycling for the polymerase chain reaction as well as real-time measurement of fluorescent signals during amplification and therefore allows for highly accurate quantification of RT-PCR products by using a kinetic, rather than endpoint readout. The relative quantity of ADARB2 cDNAs from the frontal and temporal cortices of AD patients and age-matched control individuals respectively, were determined in a number of four up to nine tissues per Braak stage.

First, a standard curve was generated to determine the efficiency of the PCR with specific primers for the gene coding for ADARB2: Primer A, SEQ ID NO: 13, 5'-GAGTGTGCAATGTTTGGACGA-3' (nucleotides 2671-2691 of SEQ ID NO: 5) and Primer B, SEQ ID NO: 14, 3'-GCACACGCACCGTTGAGTT-5' (nucleotides 2764-2782 of SEQ ID NO: 5).

PCR amplification (95°C and 1 sec, 56°C and 5 sec, and 72°C and 5 sec) was performed in a volume of 20 μl containing LightCycler-FastStart DNA Master SYBR Green I mix (contains FastStart Taq DNA polymerase, reaction buffer, dNTP mix with dUTP instead of dTTP, SYBR Green I dye, and 1 mM MgCl2; Roche), 0.5 μM primers, 2 μl of a cDNA dilution series (final concentration of 40, 20, 10, 5, 1 and 0.5 ng human total brain cDNA; Clontech) and additional 3 mM MgCl2. Melting curve analysis revealed a single peak at approximately 87.5°C with no visible primer dimers. Quality and size of the qPCR product were determined applying the DNA 500 LabChip system using the 2100 Bioanalyzer (Agilent Technologies). A single peak at the expected size of 112 bp for the gene coding for ADARB2 protein was observed in the electropherogram of the sample.

In an analogous manner, the qPCR protocol was applied to determine the PCR efficiency of cyclophilin B, using the specific primers SEQ ID NO: 15, 5'-ACTGAAGCACTACGGGCCTG-3' and SEQ ID NO: 16, 5'-AGCCGTTGGT-GTCTTTGCC-3' except for $MgCl_2$ (an additional 1 mM was added instead of 3 mM). Melting curve analysis revealed a single peak at approximately 87°C with no visible primer dimers. Bioanalyzer analysis of the PCR product showed one single peak of the expected size (62 bp).

For calculation of the standard values, first the logarithm of the used cDNA concentration was plotted against the threshold cycle value Ct for ADARB2 and Cyclophilin B respectively. The slopes and the intercepts of the standard curves (i.e. linear regressions) were calculated. In a second step, mRNA expression from frontal and inferior temporal cortices of controls and AD patients were analyzed in parallel. The Ct values were measured and converted to ng total brain cDNA using the corresponding standard curves:

$$10 \wedge (\text{Ct value} - \text{intercept}) / \text{slope} \quad [\text{ng total brain cDNA}]$$

Calculated cDNA concentration values were normalized to Cyclophilin B that was analyzed in parallel for each tested tissue probe, thus resulting values are defined as arbitrary relative expression levels. The results of such quantitative RT-PCR analysis for the gene coding for ADARB2 protein are shown in Figure 2. (vii) Statistical analysis of the mRNA expression comparing donor groups with different Braak stages.

For this analysis it was proven that absolute values of real-time quantitative PCR (Lightcycler method) between different experiments at different time points are consistent enough to be used for quantitative comparisons without usage of calibrators. Cyclophilin was used as a standard for normalization in any of the qPCR experiments for more than 100 tissues. Between others it was found to be the most consistently expressed housekeeping gene in the normalization experiments. Therefore a proof of concept was done by using values that were generated for cyclophilin.

First analysis used cyclophilin values from qPCR experiments of frontal cortex and inferior temporal cortex tissues from three different donors. From each tissue the same cDNA preparation was used in all analyzed experiments. Within this analysis no normal distribution of values was achieved due to small number of data. Therefore the method of median and its 98 %-confidence level was applied (Sachs L (1988) Statistische Methoden: Planung und Auswertung. Heidelberg New York, p. 60). This analysis revealed a middle deviation of 8.7 % from the median for comparison of absolute values and a middle deviation of 6.6 % from the median for relative comparison.

Second analysis used cyclophilin values from qPCR experiments of frontal cortex and inferior temporal cortex tissues from two different donors each, but different cDNA preparations from different time points were used. This analysis revealed a middle deviation of 29.2 % from the median for comparison of absolute values and a middle deviation of 17.6 % from the median for relative comparison. From this analysis it was concluded, that absolute values from qPCR experiments can be used, but the middle deviation from median should be taken into further considerations.

A detailed analysis of absolute values for ADARB2 was performed using the method of median and its 98 %-confidence level. Because in contrast to the mean the calculation of the median is not affected by single data outliers; therefore latter is the method of choice for a small number of data that are distributed non-normal and/or assymetric (Sachs L (1988) Statistische Methoden: Planung und Auswertung. Heidelberg New York, p. 60). Therefore, absolute levels of ADARB2 were used after relative normalization with cyclophilin. The median as well as the 98 %-confidence level was calculated for a group consisting of low level Braak stages (Braak 0 - Braak 1) and the group consisting of high level Braak stages (Braak 2 - Braak 4). The analysis was aimed to identify early onset of mRNA expression differences within the course of AD pathology. Said analysis described above is shown in Figure 3.

(viii) Verification of differential expression of the ADARB2 gene and association with AD at the protein level applying immunohistochemical analyses:

For immunofluorescence staining of ADARB2 in human brain, and for the comparison of AD-affected tissues with control tissues, post-mortem fresh-frozen frontal and temporal forebrain specimens from donors comprising patients with clinically diagnosed and neuropathologically confirmed AD at various stages of neurofibrillary pathology according to Braak and Braak (herein before and after briefly called "Braak stages") as well as age-matched non AD control individuals with neither clinical nor neuropathological signs of AD were cut at 14 μm thickness using a cryostat (Leica CM3050S). The tissue sections were air-dried at room temperature and fixed in acetone for 10 min, and air-dried again. After washing in PBS, the sections were pre-incubated with 10% normal donkey serum in phosphate buffered saline (PBS) for 30 min and then incubated with affinity-purified anti-ADARB2 goat polyclonal antiserum sc-10014 (Santa Cruz) diluted 1:15 in blocking buffer (1% bovine serum albumin in PBS), overnight at 4°C. After rinsing three times in PBS, the sections were incubated with AlexaFluor-488-conjugated donkey anti-goat IgG antiserum (Jackson/Dianova, Hamburg, Germany), in a 1:1500 dilution in blocking buffer for 2 hours at room temperature and then again washed with PBS. Simultaneous staining of neuronal somata (including nuclei) was performed as described above using a mouse monoclonal antibody against the neuron-specific marker protein NeuN (Chemicon, Hampshire, UK; dilution 1:350), followed by a secondary Cy3-conjugated donkey anti-mouse antibody (Jackson/Dianova; dilution 1:1000). Staining of nuclei was performed by incubation of the sections with 0.5 μM DAPI in PBS for 3 min. In order to block lipofuscin autofluoresence, the sections were treated with the lipophilic black dye Sudan Black B (1% w/v) in 70% ethanol for 5 min at room temperature and then sequentially dipped in 70% ethanol, destilled water and PBS. The sections were coverslipped with ProLong-Gold antifade mounting medium (Invitrogen/Molecular Probes, Karlsruhe, Germany). Microscopic epifluorescence images were obtained using an upright microscope with a mercury arc lamp (BX51, Olympus, Hamburg, Germany). The appropriate dichromic filter and mirror combinations (hereinafter called "channels") were used for the specific excitation of either fluorochrome (AlexaFluor-488, Cy3, DAPI) and for reading out the emitted fluorescence light resulting from the specific labeling by said antibodies or the nuclear DAPI stain. Microscopic images were digitally captured with a charge-coupled display camera and the appropriate image acquisiton and processing software (ColorView-II and AnalySIS, Olympus Soft Imaging Solutions GmbH, Münster, Germany). Fluorescence micrographs obtained from the different channels were overlaid in order to generate simultaneous views of the above specified immunolabelings and nuclei (DAPI) in the RGB mode, e.g. for analyzing the potential co-localization of signals from the three different channels.

(ix) Generation of cell lines inducibly expressing ADARB2:

The tet-repressor encoded on the pcDNA6/TR-vector (purchased from Invitrogen #K1020-01) was transfected into the H4-neuroglioma cell line expressing the Swedish mutant amyloid precursor protein (APP) and clonal cell lines were isolated after the addition of the antibiotic blasticidin. The resulting cell line was then used to introduce ADARB2 under the control of the tet-operator sequence into the cells. Expression of ADARB2 can be induced by the addition of 1μg/ml tetracycline following the manufacturer's instructions (Invitrogen #K1020-01).

**Claims**

1. An in vitro method of diagnosing Alzheimer's disease in a subject, which method comprises the steps of:

    (a) determining a level of (i) a transcription product of the gene coding for. ADARB2 proteins (formerly denoted as ADARB), and/or (ii) a translation product of the gene coding for ADARB2 proteins,
    (b) comparing said level of said transcription product and/or said translation product to a reference value representing a disease status of Alzheimer's disease and/or representing a known health status and/or representing a known Braak stage,
    (c) analysing whether said level is increased compared to a reference value representing a known health status, and/or is similar or equal to a reference value representing a disease status of Alzheimer's disease or representing a known Braak stage which is an indication that said subject has Alzheimer's disease.

2. The method according to claim 1 wherein said gene coding for ADARB2 proteins is the gene coding for a ADARB2 protein having SEQ ID NO: 1, or SEQ ID NO: 2, or SEQ ID NO: 3, or SEQ ID NO: 4 and wherein said translation product of the gene coding for ADARB2 proteins is the ADARB2 protein having SEQ ID NO: 1, or SEQ ID NO: 2, or SEQ ID NO: 3, or SEQ ID NO: 4.

3. Use of a kit for in vitro diagnosing Alzheimer's disease, in a subject, according to the method of claim 1, wherein the kit comprises at least one reagent which is selected from the group consisting of reagents that detect (i) a transcription product of the gene coding for ADARB2 proteins and/or (ii) a translation product of the gene coding for ADARB2 proteins.

4. The use of claim 3 wherein the ADARB2 proteins have the SEQ ID NO: 1, or SEQ ID NO: 2, or SEQ ID NO: 3, or SEQ ID NO: 4.

5. A genetically modified non-human animal comprising a non-native gene sequence coding for a ADARB2 protein under the control of a transcriptional element which is not the native ADARB2 gene transcriptional control element, wherein the expression, disruption or alteration of said gene sequence results in said non-human animal exhibiting a predisposition to developing signs of Alzheimer's disease, and wherein the animal comprises a non-native gene sequence coding for ADARB2 proteins wherein the ADARB2 proteins have the SEQ ID NO: 1, or SEQ ID NO: 2, or SEQ ID NO: 3, or SEQ ID NO: 4..

6. The animal according to claim 5 wherein said signs comprise the formation of neurofibrillary tangles and/or wherein said animal is an insect or a rodent.

7. The use of a genetically modified non-human animal according to one of claims 5 to 6 as a non-human test animal and/or control animal for screening, testing, and/or validating compounds, agents, and modulators in the development of diagnostics and therapeutics useful for the treatment of Alzheimer's disease.

8. The in vitro use of a cell in which a gene sequence coding for a ADARB2 protein is expressed, disrupted, or altered for screening, testing, and/or validating compounds, agents, and modulators in the development of diagnostics and therapeutics useful for the treatment of Alzheimer's disease, wherein the ADARB2 protein has SEQ ID NO: 1, or SEQ ID NO: 2, or SEQ ID NO: 3, or SEQ ID NO: 4..

9. The use of a cell according to claim 8 wherein the expression, disruption, or alteration of said gene sequence results in said cell exhibiting a predisposition to developing signs of Alzheimer's disease.

10. An in vitro method of screening for agents, modulators, antagonists or agonists for use in the treatment or prevention of Alzheimer's disease, which agents, modulators, antagonists or agonists have an ability to alter expression or level of one or more substances selected from the group consisting of

    (i) a gene coding for ADARB2 proteins, and/or
    (ii) a transcription product of the gene coding for ADARB2 proteins, and/or
    (iii) a translation product of the gene coding for ADARB2 proteins,
    said method comprises:

        (a) contacting a cell with a test compound;

(b) measuring the level and/or expression of one or more substances recited in (i) to (iii);
(c) measuring the level and/or expression of one or more substances recited in (i) to (iii) in a control cell not contacted with said test compound; and

comparing the levels and/or expression of the substances in the cells of step (b) and (c), wherein an alteration in the level and/or expression of the substances in the contacted cells indicates that the test compound is an agent, modulator, or antagonist or agonist for use in the treatment of Alzheimer's disease.

**11.** The method of claim 10 wherein the ADARB2 proteins have the SEQ ID NO: 1, or SEQ ID NO: 2, or SEQ ID NO: 3, or SEQ ID NO: 4.

**12.** An in vitro use of a protein molecule being a translation product of the gene coding for ADARB2 as diagnostic target for detecting Alzheimer's disease or as screening target for modulators, agents or compounds preventing, or treating, or ameliorating Alzheimer's disease.

**13.** The use according to claim 12 wherein the ADARB2 proteins have SEQ ID NO: 1, or SEQ ID NO: 2, or SEQ ID NO: 3, or SEQ ID NO: 4.

**14.** An in vitro use of an antibody specifically immunoreactive with an immunogen, wherein said immunogen is a translation product of a gene coding for ADARB2 proteins for detecting the pathological state of a cell in a sample obtained from a subject, comprising immunocytochemical staining of said cell with said antibody, wherein an altered degree of staining, or an altered staining pattern in said cell compared to a cell representing a known health status indicates a pathological state of said cell which relates to Alzheimer's disease.

**15.** The use of claim 14 wherein the ADARB2 proteins have SEQ ID NO: 1, or SEQ ID NO: 2, or SEQ ID NO: 3, or SEQ ID NO: 4.

**Patentansprüche**

**1.** In-vitro-Verfahren zum Diagnostizieren der Alzheimer-Krankheit bei einem Patienten, wobei das Verfahren die folgenden Schritte umfasst:

(a) Bestimmen einer Konzentration von (i) einem Transcriptionsprodukt des Gens, das für ADARB2-Proteine (früher als ADARB bezeichnet) codiert, und/oder (ii) einem Translationsprodukt des Gens, das für ADARB2-Proteine codiert;
(b) Vergleichen der Konzentration des Transcriptionsprodukts und/oder des Translationsprodukts mit einem Referenzwert, der einen Krankheitszustand der Alzheimer-Krankheit darstellt und/oder einen bekannten Gesundheitszustand darstellt und/oder ein bekanntes Braak-Stadium darstellt;
(c) Analysieren, ob die Konzentration im Vergleich zu einem Referenzwert, der einen bekannten Gesundheitszustand darstellt, erhöht ist und/oder ähnlich oder gleich ist wie ein Referenzwert, der einen bekannten Krankheitszustand der Alzheimer-Krankheit darstellt oder ein bekanntes Braak-Stadium darstellt, was darauf hinweist, dass der Patient an Alzheimer-Krankheit leidet.

**2.** Verfahren gemäß Anspruch 1, wobei das Gen, das für ADARB2-Proteine codiert, das Gen ist, das für ein ADARB2-Protein mit SEQ ID Nr. 1 oder SEQ ID Nr. 2 oder SEQ ID Nr. 3 oder SEQ ID Nr. 4 codiert, und wobei das Translationsprodukt des Gens, das für ADARB2-Proteine codiert, das ADARB2-Protein mit SEQ ID Nr. 1 oder SEQ ID Nr. 2 oder SEQ ID Nr. 3 oder SEQ ID Nr. 4 ist.

**3.** Verwendung eines Kits zum in-vitro-Diagnostizieren der Alzheimer-Krankheit bei einem Patienten gemäß dem Verfahren von Anspruch 1, wobei der Kit wenigstens ein Reagens umfasst, das aus der Gruppe ausgewählt ist, die aus Reagentien besteht, die (i) ein Transcriptionsprodukt des Gens, das für ADARB2-Proteine codiert, und/oder (ii) ein Translationsprodukt des Gens, das für ADARB2-Proteine codiert, nachweisen.

**4.** Verwendung gemäß Anspruch 3, wobei die ADARB2-Proteine die SEQ ID Nr. 1 oder SEQ ID Nr. 2 oder SEQ ID Nr. 3 oder SEQ ID Nr. 4 aufweisen.

**5.** Genetisch modifiziertes nichthumanes Tier, das eine nichtnative Gensequenz, die für ein ADARB2-Protein codiert, unter der Kontrolle eines Transcriptionselements, bei dem es sich nicht um das native Transcriptionskontrollelement ADARB2-Gens handelt, umfasst, wobei die Expression, Disruption oder Veränderung der Gensequenz dazu führt, dass das nichthumane Tier eine Prädisposition für die Entwicklung von Symptomen der Alzheimer-Krankheit aufweist, und wobei das Tier eine nichtnative Gensequenz umfasst, die für ADARB2-Proteine codiert, wobei die ADARB2-Proteine die SEQ ID Nr. 1 oder SEQ ID Nr. 2 oder SEQ ID Nr. 3 oder SEQ ID Nr. 4 aufweisen.

**6.** Tier gemäß Anspruch 5, wobei die Symptome die Bildung von neurofibrillären Tangles umfassen und/oder wobei das Tier ein Insekt oder ein Nagetier ist.

**7.** Verwendung eines genetisch modifizierten nichthumanen Tiers gemäß einem der Ansprüche 5 oder 6 als nichthumanes Versuchstier und/oder Kontrolltier zum Screening, Testen und/oder Validieren von Verbindungen, Mitteln und Modulatoren bei der Entwicklung von Diagnostika und Therapeutika, die für die Behandlung von Alzheimer-Krankheit geeignet sind.

**8.** In-vitro-Verwendung einer Zelle, in der eine für ein ADARB2-Protein codierende Gensequenz exprimiert, disrumpiert oder verändert ist, zum Screening, Testen und/oder Validieren von Verbindungen, Mitteln und Modulatoren bei der Entwicklung von Diagnostika und Therapeutika, die für die Behandlung von Alzheimer-Krankheit geeignet sind, wobei das ADARB2-Protein die SEQ ID Nr. 1 oder SEQ ID Nr. 2 oder SEQ ID Nr. 3 oder SEQ ID Nr. 4 aufweist.

**9.** Verwendung einer Zelle gemäß Anspruch 8, wobei die Expression, Disruption oder Veränderung der Gensequenz dazu führt, dass die Zelle eine Prädisposition für die Entwicklung von Symptomen der Alzheimer-Krankheit aufweist.

**10.** In-vitro-Verfahren zum Screening nach Mitteln, Modulatoren, Antagonisten oder Agonisten zur Verwendung bei der Behandlung oder Prävention von Alzheimer-Krankheit, wobei die Mittel, Modulatoren, Antagonisten oder Agonisten die Fähigkeit haben, die Expression oder die Konzentration von einer oder mehreren Substanzen zu verändern, die aus der Gruppe ausgewählt sind, die aus Folgenden besteht:

(i) einem Gen, das für ADARB2-Proteine codiert; und/oder
(ii) einem Transcriptionsprodukt des Gens, das für ADARB2-Proteine codiert; und/oder
(iii) einem Translationsprodukt des Gens, das für ADARB2-Proteine codiert;
wobei das Verfahren Folgendes umfasst:

(a) In-Kontakt-Bringen einer Zelle mit einer Testverbindung;
(b) Messen der Aktivität und/oder der Expression von einer oder mehreren Substanzen, die in (i) bis (iii) genannt sind;
(c) Messen der Aktivität und/oder der Expression von einer oder mehreren Substanzen, die in (i) bis (iii) genannt sind, in einer Kontrollzelle, die nicht mit der Testverbindung in Kontakt gebracht wurde; und

Vergleichen der Konzentrationen und/oder Expression der Substanz in den Zellen der Schritte (b) und (c), wobei eine Änderung der Aktivität und/oder der Expression der Substanzen in den kontaktierten Zellen anzeigt, dass die Testverbindung ein Mittel, Modulator oder Antagonist oder Agonist zur Verwendung bei der Behandlung der Alzheimer-Krankheit ist.

**11.** Verfahren gemäß Anspruch 10, wobei die ADARB2-Proteine die SEQ ID Nr. 1 oder SEQ ID Nr. 2 oder SEQ ID Nr. 3 oder SEQ ID Nr. 4 aufweisen.

**12.** In-vitro-Verwendung eines Proteinmoleküls, das ein Translationsprodukt des für ADARB2 codierenden Gens ist, als diagnostisches Target zum Nachweisen der Alzheimer-Krankheit oder als Screening-Target für Modulatoren, Mittel oder Verbindungen, die Alzheimer-Krankheit verhindern oder behandeln oder lindern.

**13.** Verwendung gemäß Anspruch 12, wobei die ADARB2-Proteine die SEQ ID Nr. 1 oder SEQ ID Nr. 2 oder SEQ ID Nr. 3 oder SEQ ID Nr. 4 aufweisen.

**14.** In-vitro-Verwendung eines Antikörpers, der spezifisch immunreaktiv gegenüber einem Immunogen ist, wobei das Immunogen ein Translationsprodukt eines Gens ist, das für ADARB2-Proteine codiert, zum Nachweisen des pathologischen Zustands einer Zelle in einer Probe, die von einem Patienten erhalten wurde, umfassend das immuncytochemische Anfärben der Zelle mit dem Antikörper, wobei ein veränderter Grad der Anfärbung oder ein verändertes Anfärbungsmuster in der Zelle im Vergleich zu einer Zelle, die einen bekannten Gesundheitszustand darstellt, einen pathologischen Zustand der Zelle, der sich auf die Alzheimer-Krankheit bezieht, anzeigt.

**15.** Verwendung gemäß Anspruch 14, wobei die ADARB2-Proteine die SEQ ID Nr. 1 oder SEQ ID Nr. 2 oder SEQ ID Nr. 3 oder SEQ ID Nr. 4 aufweisen.

## Revendications

**1.** Procédé *in vitro* de diagnostic de la maladie d'Alzheimer chez un sujet, lequel procédé comprend les étapes consistant à :

(a) déterminer un taux (i) d'un produit de transcription du gène codant pour les protéines ADARB2 (antérieurement désignées par ADARB) et/ou (ii) d'un produit de traduction du gène codant pour des protéines ADARB2,
(b) comparer ledit taux dudit produit de transcription et/ou dudit produit de traduction à une valeur de référence représentant un état pathologique de la maladie d'Alzheimer et/ou représentant un état de santé connu et/ou représentant un stade de Braak connu,
(c) déterminer si ledit taux est augmenté par rapport à une valeur de référence représentant un état de santé connu et/ou est similaire ou égal à une valeur de référence représentant un état pathologique de la maladie d'Alzheimer ou représentant un stade de Braak connu qui indique que ledit sujet a la maladie d'Alzheimer.

**2.** Procédé selon la revendication 1, dans lequel ledit gène codant pour des protéines ADARB2 est le gène codant pour une protéine ADARB2 ayant la SEQ ID NO : 1, ou la SEQ ID NO : 2, ou la SEQ ID NO : 3 ou la SEQ ID NO : 4 et où ledit produit de traduction du gène codant pour des protéines ADARB2 est la protéine ADARB2 ayant la SEQ ID NO : 1, ou la SEQ ID NO : 2, ou la SEQ ID NO : 3 ou la SEQ ID NO : 4.

**3.** Utilisation d'un kit pour diagnostiquer *in vitro* la maladie d'Alzheimer, chez un sujet, selon le procédé de la revendication 1, dans lequel le kit comprend au moins un réactif qui est choisi dans le groupe constitué par les réactifs qui détectent (i) un produit de transcription du gène codant pour des protéines ADARB2 et/ou (ii) un produit de traduction du gène codant pour des protéines ADARB2.

**4.** Utilisation selon la revendication 3, dans laquelle les protéines ADARB2 ont la SEQ ID NO : 1, ou la SEQ ID NO : 2, ou la SEQ ID NO : 3 ou la SEQ ID NO : 4.

**5.** Animal non humain génétiquement modifié comprenant une séquence de gènes non native codant pour une protéine ADARB2 sous la régulation d'un élément transcriptionnel qui n'est pas l'élément de régulation transcriptionnel de gène ADARB2 natif, où l'expression, la rupture ou la modification de ladite séquence de gènes conduit ledit animal non humain à montrer une prédisposition à développer des signes de la maladie d'Alzheimer, et où l'animal comprend une séquence de gènes non native, codant pour des protéines ADARB2 où les protéines ADARB2 ont la SEQ ID NO : 1, ou la SEQ ID NO : 2, ou la SEQ ID NO : 3 ou la SEQ ID NO : 4.

**6.** Animal selon la revendication 5, dans lequel lesdits signes comprennent la formation de dégénérescences neurofibrillaires et/ou dans lequel ledit animal est un insecte ou un rongeur.

**7.** Utilisation d'un animal non humain génétiquement modifié selon l'une des revendications 5 à 6, en tant qu'animal d'essai non humain et/ou animal témoin pour cribler, évaluer et/ou valider des composés, agents et modulateurs dans le développement de diagnostics et agents thérapeutiques utiles pour le traitement de la maladie d'Alzheimer.

**8.** Utilisation *in vitro* d'une cellule dans laquelle une séquence de gènes codant pour une protéine ADARB2 est exprimée, rompue ou modifiée pour cribler, évaluer et/ou valider des composés, agents et modulateurs dans le développement de diagnostics et agents thérapeutiques utiles pour le traitement de la maladie d'Alzheimer, où la protéine ADARB2 a la SEQ ID NO : 1, ou la SEQ ID NO : 2, ou la SEQ ID NO : 3 ou la SEQ ID NO : 4.

**9.** Utilisation d'une cellule selon la revendication 8, dans laquelle l'expression, la rupture ou la modification de ladite séquence de gènes conduit ladite cellule à montrer une prédisposition à développer des signes de la maladie d'Alzheimer.

**10.** Procédé *in vitro* de criblage d'agents, de modulateurs d'antagonistes ou d'agonistes à utiliser dans le traitement ou la prévention de la maladie d'Alzheimer, lesquels agents, modulateurs, antagonistes ou agonistes ont la capacité de modifier l'expression ou le taux d'une ou plusieurs substances choisies dans le groupe consistant en

(i) un gène codant pour des protéines ADARB2, et/ou
(ii) un produit de transcription du gène codant pour des protéines ADARB2, et/ou
(iii) un produit de traduction du gène codant pour des protéines ADARB2,
ledit procédé comprenant les étapes consistant à :

(a) mettre en contact une cellule avec un composé d'essai ;
(b) mesurer le taux et/ou l'expression d'une ou plusieurs substances citées dans (i) à (iii) ;
(c) mesurer le taux et/ou l'expression d'une ou plusieurs substances citées dans (i) à (iii) dans une cellule témoin non mise en contact avec ledit composé d'essai ; et

comparer les taux et/ou l'expression des substances dans les cellules de l'étape (b) et (c), où une modification du taux et/ou de l'expression des substances dans les cellules mises en contact indiquent que le composé d'essai est un agent, modulateur, ou antagoniste ou agoniste à utiliser dans le traitement de la maladie d'Alzheimer.

**11.** Procédé selon la revendication 10, dans lequel les protéines ADARB2 ont la SEQ ID NO : 1, ou la SEQ ID NO : 2, ou la SEQ ID NO : 3 ou la SEQ ID NO : 4.

**12.** Utilisation *in vitro* d'une molécule de protéine qui est un produit de traduction du gène codant pour ADARB2 comme cible diagnostique pour détecter la maladie d'Alzheimer ou comme cible de criblage pour des modulateurs, agents ou composés prévenant, ou traitant, ou améliorant la maladie d'Alzheimer.

**13.** Utilisation selon la revendication 12, dans laquelle les protéines ADARB2 ont la SEQ ID NO : 1, ou la SEQ ID NO : 2, ou la SEQ ID NO : 3 ou la SEQ ID NO : 4.

**14.** Utilisation *in vitro* d'un anticorps spécifiquement immunoréactif avec un immunogène, dans laquelle ledit immunogène est un produit de traduction d'un gène codant pour des protéines ADARB2 pour détecter l'état pathologique d'une cellule dans un échantillon obtenu à partir d'un sujet, comprenant la coloration immunocytochimique de ladite cellule avec ledit anticorps, où un degré modifié de coloration, ou un motif de coloration modifié dans ladite cellule par rapport à une cellule représentant un état de santé connu indique un état pathologique de ladite cellule qui concerne la maladie d'Alzheimer.

**15.** Utilisation selon la revendication 14, dans laquelle les protéines ADARB2 ont la SEQ ID NO : 1, ou la SEQ ID NO : 2, ou la SEQ ID NO : 3 ou la SEQ ID NO : 4.

Fig. 1: Identification of the differentially expressed gene ADARB2 in genechip microarray analysis

Expression level in frontal cortex

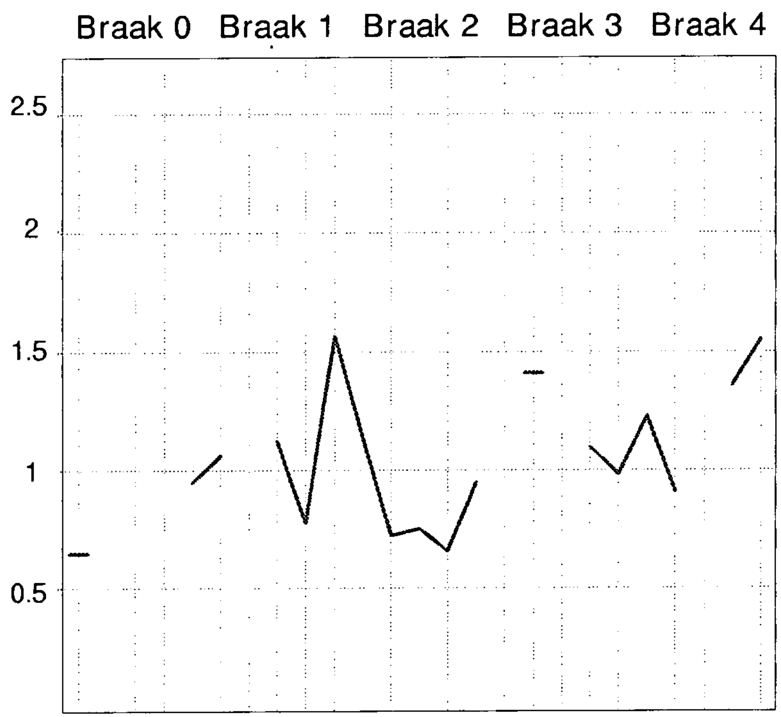


Expression level in inferior temporal cortex

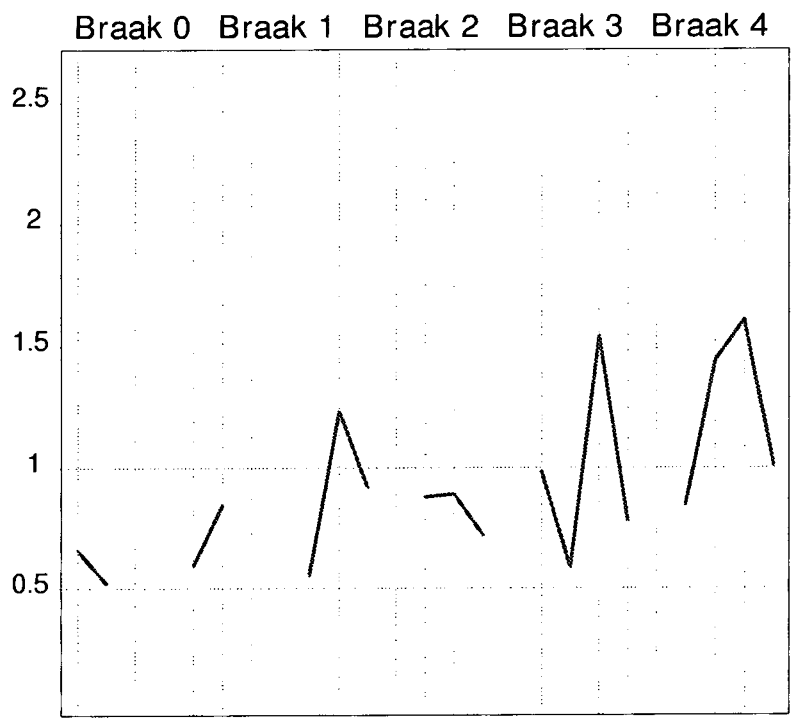

Fig. 2: Analysis of ADARB2 mRNA expression by quantitative PCR

measured data of ADARB2 mRNA expression in frontal and inferior temporal cortices in Braak stages 0 – 4

| Donor | Braak stage | Frontal | Temporal |
|---|---|---|---|
| C011 | 0 | 14.1684 | 12.4814 |
| C012 | 0 | 14.4335 | 17.6409 |
| C026 | 0 | 9.2747 | 5.5663 |
| C027 | 0 | 1.3850 | 3.0245 |
| C032 | 0 | 18.5683 | 17.5738 |
| C014 | 1 | 39.4103 | 29.4609 |
| C028 | 1 | 7.8876 | 6.4351 |
| C029 | 1 | 23.1376 | 23.3497 |
| C030 | 1 | 18.2787 | 12.8517 |
| C036 | 1 | 32.2145 | 46.6548 |
| C038 | 1 | 35.3490 | 43.3032 |
| C039 | 1 | 21.9469 | 14.7987 |
| C008 | 2 | 13.1023 | 29.6996 |
| C031 | 2 | 11.5479 | 21.4060 |
| C033 | 2 | 159.1542 | 126.3843 |
| C034 | 2 | 110.4463 | 84.8242 |
| DE03 | 2 | 24.0244 | 28.6351 |
| C025 | 3 | 42.9673 | 29.3772 |
| DE07 | 3 | 19.5922 | 41.2197 |
| DE11 | 3 | 36.8783 | 31.6719 |
| C057 | 3 | 25.5563 | 19.9531 |
| P012 | 4 | 30.7312 | 34.7085 |
| P046 | 4 | 29.4983 | 26.9071 |
| P047 | 4 | 25.8196 | 37.2768 |
| P068 | 4 | 30.6652 | 29.2728 |

Fig. 3: Statistical analysis of differential ADARB2 mRNA expression

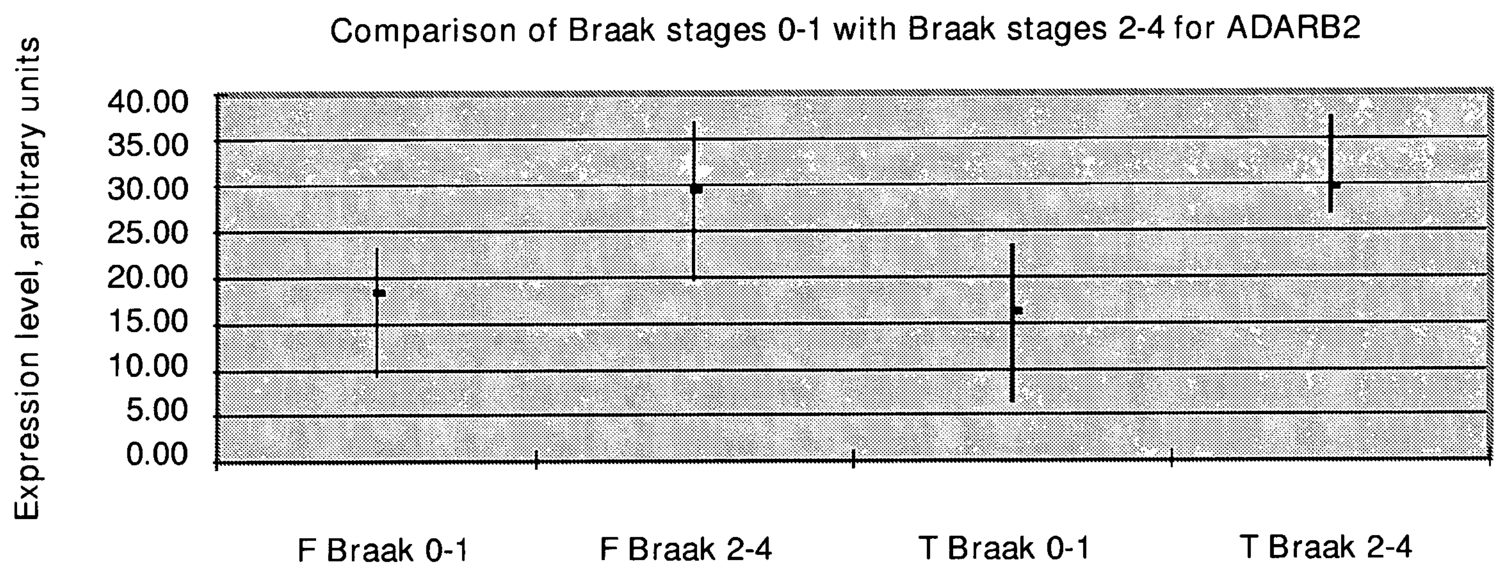

Fig. 4 : A) SEQ ID NO: 1,
amino acid sequence of
human ADARB2 sv1 protein

Length: 739 aa

| | | | | | |
|---|---|---|---|---|---|
| 1 | MASVLGSGRG | SGGLSSQLKC | KSKRRRRRRS | KRKDKVSILS | TFLAPFKHLS |
| 51 | PGITNTEDDD | TLSTSSAEVK | ENRNVGNLAA | RPPPSGDRAR | GGAPGAKRKR |
| 101 | PLEEGNGGHL | CKLQLVWKKL | SWSVAPKNAL | VQLHELRPGL | QYRTVSQTGP |
| 151 | VHAPVFAVAV | EVNGLTFEGT | GPTKKKAKMR | AAELALRSFV | QFPNACQAHL |
| 201 | AMGGGPGPGT | DFTSDQADFP | DTLFQEFEPP | APRPGLAGGR | PGDAALLSAA |
| 251 | YGRRRLLCRA | LDLVGPTPAT | PAAPGERNPV | VLLNRLRAGL | RYVCLAEPAE |
| 301 | RRARSFVMAV | SVDGRTFEGS | GRSKKLARGQ | AAQAALQELF | DIQMPGHAPG |
| 351 | RARRTPMPQE | FADSISQLVT | QKFREVTTDL | TPMHARHKAL | AGIVMTKGLD |
| 401 | ARQAQVVALS | SGTKCISGEH | LSDQGLVVND | CHAEVVARRA | FLHFLYTQLE |
| 451 | LHLSKRREDS | ERSIFVRLKE | GGYRLRENIL | FHLYVSTSPC | GDARLHSPYE |
| 501 | ITTDLHSSKH | LVRKFRGHLR | TKIESGEGTV | PVRGPSAVQT | WDGVLLGEQL |
| 551 | ITMSCTDKIA | RWNVLGLQGA | LLSHFVEPVY | LQSIVVGSLH | HTGHLARVMS |
| 601 | HRMEGVGQLP | ASYRHNRPLL | SGVSDAEARQ | PGKSPPFSMN | WVVGSADLEI |
| 651 | INATTGRRSC | GGPSRLCKHV | LSARWARLYG | RLSTRTPSPG | DTPSMYCEAK |
| 701 | LGAHTYQSVK | QQLFKAFQKA | GLGTWVRKPP | EQQQFLLTL | |

Fig. 4 : B) SEQ ID NO: 2,
amino acid sequence of
human ADARB2 sv2 protein

Length: 248 aa

```
  1  MGLWSPVPGN SVPRAPVMAG LGLGHSQGAP QGPHDGKTDR SSGPGRRRTE
 51  RTRLPGLLGG APLTAGSPHR WNVLGLQGAL LSHFVEPVYL QSIVVGSLHH
101  TGHLARVMSH RMEGVGQLPA SYRHNRPLLS GVSDAEARQP GKSPPFSMNW
151  VVGSADLEII NATTGRRSCG GPSRLCKHVL SARWARLYGR LSTRTPSPGD
201  TPSMYCEAKL GAHTYQSVKQ QLFKAFQKAG LGTWVRKPPE QQQFLLTL
```

Fig. 4 : C) SEQ ID NO: 3,
amino acid sequence of
human ADARB2 sv3 protein

Length: 141 aa

```
  1  MSHRMEGVGQ LPASYRHNRP LLSGVSDAEA RQPGKSPPFS MNWVVGSADL
 51  EIINATTGRR SCGGPSRLCK HVLSARWARL YGRLSTRTPS PGDTPSMYCE
101  AKLGAHTYQS VKQQLFKAFQ KAGLGTWVRK PPEQQQFLLT L
```

Fig. 4 : D) SEQ ID NO: 4,
amino acid sequence of
human ADARB2 sv4 protein

Length: 134 aa

```
  1  MNERETPGQL GLGNASGVSD AEARQPGKSP PFSMNWVVGS ADLEIINATT
 51  GRRSCGGPSR LCKHVLSARW ARLYGRLSTR TPSPGDTPSM YCEAKLGAHT
101  YQSVKQQLFK AFQKAGLGTW VRKPPEQQQF LLTL
```

Fig. 5 : A) SEQ ID NO: 5,
nucleotide sequence of
human ADARB2 sv1 cDNA

Length: 3606 bp

```
   1  TGCGCTGGGA GCCCAGAGCA GGAGGCAGAG CCCGCGGGAA GCTCGGAGCA
  51  CTCAGGACGC CGCGCGCCCT TCCCCGCCCT CCCTGACCAG GGAGCAGCTC
 101  GCTCCAGGCG CCCAGCCGAG GCCCCCAGCC CAGTGGGAGC AAGTCATAGA
 151  GAACAATTCG AGAGACAGAG AGACGGAGCG CGCTTTCCTG CTCAGTCCTG
 201  AAAAGTGAGC CGCTCCCGGG TTTGCAACCT CAAGCTTCGC AGCAGCGGCG
 251  GCGGCGGCTG CCGGGAAGGA GGCAGGTGCA GGTGCAGGAG GGAGGCGGCT
 301  CTGGGCTCCG CGCCTGGGTC TCGGCCATGG CCTCGGTCCT GGGGAGCGGC
 351  AGAGGGTCTG GAGGGCTGAG CAGTCAACTC AAATGCAAGT CCAAGAGGAG
 401  GAGGAGGCGG AGGTCCAAGC GGAAAGATAA AGTAAGCATA TTGTCAACCT
 451  TCCTCGCTCC TTTCAAGCAC CTGAGTCCTG GCATCACAAA CACGGAGGAT
 501  GACGACACCC TCAGTACCAG CAGCGCGGAG GTGAAGGAGA ACCGCAACGT
 551  GGGCAACCTG GCCGCGCGGC CACCGCCCTC CGGGGACCGG GCCCGGGGCG
 601  GCGCGCCCGG CGCGAAGAGG AAGCGGCCGC TGGAGGAGGG GAATGGGGGC
 651  CACTTGTGCA AACTGCAGCT GGTCTGGAAG AAGCTGTCGT GGTCGGTGGC
 701  GCCCAAGAAC GCGCTGGTGC AGCTGCACGA GCTGAGGCCG GGCCTGCAGT
 751  ACCGGACAGT GTCGCAGACG GGCCCGGTGC ATGCCCCGGT CTTCGCGGTA
 801  GCGGTGGAGG TGAACGGGCT CACGTTCGAG GGCACAGGCC CCACCAAGAA
 851  GAAGGCCAAG ATGCGCGCGG CGGAGCTGGC ACTCAGGTCC TTCGTGCAGT
 901  TCCCCAACGC CTGCCAGGCG CACCTGGCCA TGGGCGGGGG CCCGGGCCCC
 951  GGCACGGACT TCACCTCCGA CCAGGCCGAT TTCCCCGACA CGCTCTTCCA
1001  GGAGTTCGAG CCCCCGGCGC CGCGCCCCGG ACTCGCGGGA GGCCGCCCCG
1051  GGGACGCCGC GCTTCTGTCC GCGGCCTACG GGCGACGGCG GCTGCTGTGC
1101  CGCGCGCTGG ACCTGGTGGG CCCGACCCCC GCCACCCCCG CGGCCCCGGG
1151  CGAGCGCAAC CCCGTGGTGC TGCTGAACCG CCTGCGCGCC GGGCTGCGCT
1201  ACGTGTGTCT GGCAGAACCG GCCGAGCGGC GCGCGCGGAG CTTCGTGATG
1251  GCCGTGAGCG TGGACGGCAG GACGTTCGAG GGCTCGGGGC GCAGCAAGAA
1301  GCTGGCCCGG GGTCAGGCCG CGCAGGCCGC ACTGCAGGAG CTGTTCGACA
1351  TCCAGATGCC CGGCCACGCG CCCGGCAGGG CCAGGAGGAC GCCAATGCCG
1401  CAGGAATTCG CAGACTCCAT ATCCCAGCTG GTCACACAGA AGTTCCGCGA
1451  GGTGACGACG GACCTCACGC CCATGCACGC CCGCCATAAA GCGCTGGCAG
1501  GAATCGTCAT GACCAAAGGC CTGGATGCTC GGCAGGCGCA GGTCGTGGCC
1551  CTGTCCTCGG GGACCAAGTG CATCAGCGGC GAGCACCTCA GTGACCAGGG
1601  GCTGGTGGTG AATGACTGCC ACGCGGAGGT CGTGGCCCGG CGGGCGTTCC
1651  TGCACTTCCT CTACACGCAG CTGGAGCTGC ACCTGAGCAA GCGGCGCGAG
1701  GACTCAGAGC GATCGATATT CGTGCGGTTA AAAGAAGGTG GCTACCGGCT
1751  GCGAGAGAAC ATCCTCTTCC ATCTCTACGT GAGCACCTCC CCCTGTGGAG
1801  ACGCAAGACT CCACTCTCCC TACGAGATCA CCACAGACCT GCACAGCAGC
1851  AAACACCTCG TCAGGAAGTT CCGCGGGCAC CTGCGCACCA AGATCGAGTC
1901  CGGGGAAGGG ACGGTCCCCG TGCGTGGCCC CAGCGCAGTG CAGACCTGGG
1951  ACGGCGTCCT GCTGGGGGAG CAGCTGATCA CCATGTCCTG CACGGACAAG
2001  ATCGCCAGGT GGAACGTCCT GGGGCTGCAG GGCGCGCTCC TGTCCCACTT
2051  CGTGGAGCCC GTGTACCTGC AGAGCATCGT GGTGGGCAGC CTGCACCACA
2101  CGGGCCACCT CGCACGCGTC ATGAGCCACC GCATGGAGGG TGTCGGCCAG
2151  CTGCCCGCCT CCTACCGGCA CAACCGGCCT CTCCTCAGCG GCGTGAGTGA
2201  CGCCGAGGCG CGCCAGCCGG GGAAGTCGCC CCCCTTCAGC ATGAACTGGG
```

2251 TCGTGGGCAG CGCGGACCTG GAGATTATCA ACGCCACCAC TGGGCGGAGG
2301 AGCTGTGGGG GCCCATCCCG GCTCTGCAAG CACGTGCTGT CTGCACGGTG
2351 GGCGCGGCTG TATGGCAGGC TGAGCACACG GACACCCAGC CCTGGAGACA
2401 CGCCCTCCAT GTACTGTGAG GCCAAGCTGG GGGCGCACAC CTACCAGTCT
2451 GTGAAACAGC AGCTGTTCAA GGCCTTTCAG AAGGCTGGCC TGGGCACCTG
2501 GGTGAGGAAA CCACCGGAGC AGCAGCAGTT TCTACTGACT CTCTAGGCTG
2551 CGGGCTCCTG GCTGCTGGAG CTGAGCGGGA CGCTGGAGGG ATGGGACCGT
2601 GTCTGGGGGG CGACGTGGCG GGTCGGCCGG TTCCCTGCAT TCGTTTTACT
2651 TTGGTGTCCC AGAAACACGC GAGTGTGCAA TGTTTGGACG AGCAACAACA
2701 CAAATTCAGA ACGTGCCTCT TTCCAGATCG CTGGCCCCAG AACCCTGTCC
2751 CCCACACCCA GGGGCACATG CACTGTTGAG TTAGCGCCGA CTCTTCCTGT
2801 GGAGTCTGAG GGAGGGGCTC CATTCAGGCA AAGGGGTTTT AGCTGCAGCC
2851 TTGGAAGGAG GCACCGACAC GACACCAGGC AGGAGTGAGC CTCAGGCCCC
2901 GTCCCTGCAC CCCACCCCTG CGTGCGCCTC TTGGTGATGC TGGGGTCTCA
2951 CTAGCTTGAG GGGGCACATG AAGATAAGCC ACAAATGAAG AGAAAAGCCA
3001 TGCCCACCCC AGCCCCAGAG AAACCAATAA GAATCCTCTA TTATTTTCAC
3051 TATTCATTTA GGTTTTTATA CTCCACCTCC TTTCAAAAAA GATTTAAGAT
3101 GTACGACATT ACCGAACACC TAAAATAGAA CCAGAGAAAC GAAAGCCATT
3151 CCCACAAAGT GAAGGAACAG TTTCCAAAAC CCCTGCGAGG CAGAGTTAAG
3201 ACCTGTGACG TGGGGAGCCT GGAAACACCC GGCCCACGCG TGCTGCAGAT
3251 GTGGGGAGCC TGGAAACACC CGGCCCACGC GTGCTGCAGA TGTGGGGAGC
3301 CTGGAAACAC CCGGCCCACG CGTGCTGCAG ATGTGGGGAG TCTGGAAACA
3351 CCCGGCCCAC GCGTGCTGCA GATGTGGGGA GCCCGGAAAC ACCTGGCCCA
3401 TGCCTGTTCA GTCCGTCATG GCGTTTGGTT TATGGTGGCC GATACTAAGA
3451 GCCACTGAGG TCTGGGTTGT GTAAGGGTTA GCTACCACCC TATAACCTCC
3501 CTACCAGGTA ATAAGGAGAC CAAGTCTTTG ATCAAATTCT GATTTTTTAA
3551 CTAAATTTTC ATTAGAAAGC AAGCACAAAA TAAAATCAAA ACAACCTTTA
3601 CCTTCA

Fig. 5 : B) SEQ I D NO: 6, nucleotide sequence of human ADARB2 sv2 cDNA

Length: 1810 bp

```
   1  ACACATGGGC CTGTGGAGCC CAGTCCCAGG CAACAGTGTC CCCAGAGCCC
  51  CAGTGATGGC TGGGTTGGGT CTGGGGCACT CCCAGGGAGC TCCCCAAGGT
 101  CCGCACGATG GGAAGACTGA CAGGTCCTCA GGACCAGGCC GCAGACGGAC
 151  AGAGAGGACC AGGCTCCCTG GGCTCCTGGG TGGGGCTCCG CTGACTGCTG
 201  GCTCTCCCCA CAGGTGGAAC GTCCTGGGGC TGCAGGGCGC GCTCCTGTCC
 251  CACTTCGTGG AGCCCGTGTA CCTGCAGAGC ATCGTGGTGG GCAGCCTGCA
 301  CCACACGGGC CACCTCGCAC GCGTCATGAG CCACCGCATG GAGGGTGTCG
 351  GCCAGCTGCC CGCCTCCTAC CGGCACAACC GGCCTCTCCT CAGCGGCGTG
 401  AGTGACGCCG AGGCGCGCCA GCCGGGGAAG TCGCCCCCCT TCAGCATGAA
 451  CTGGGTCGTG GGCAGCGCGG ACCTGGAGAT TATCAACGCC ACCACTGGGC
 501  GGAGGAGCTG TGGGGGCCCA TCCCGGCTCT GCAAGCACGT GCTGTCTGCA
 551  CGGTGGGCGC GGCTGTATGG CAGGCTGAGC ACACGGACAC CCAGCCCTGG
 601  AGACACGCCC TCCATGTACT GTGAGGCCAA GCTGGGGGCG CACACCTACC
 651  AGTCTGTGAA ACAGCAGCTG TTCAAGGCCT TTCAGAAGGC TGGCCTGGGC
 701  ACCTGGGTGA GGAAACCACC GGAGCAGCAG CAGTTTCTAC TGACTCTCTA
 751  GGCTGCGGGC TCCTGGCTGC TGGAGCTGAG CGGGACGCTG GAGGGATGGG
 801  ACCGTGTCTG GGGGGCGACG TGGCGGGTCG GCCGGTTCCC TGCATTCGTT
 851  TTACTTTGGT GTCCCAGAAA CACGCGAGTG TGCAATGTTT GGACGAGCAA
 901  CAACACAAAT TCAGAACGTG CCTCTTTCCA GATCGCTGGC CCCAGAACCC
 951  TGTCCCCCAC ACCCAGGGGC ACATGCACTG TTGAGTTAGC GCCGACTCTT
1001  CCTGTGGAGT CTGAGGGAGG GGCTCCATTC AGGCAAAGGG GTTTTAGCTG
1051  CAGCCTTGGA AGGAGGCACC GACACGACAC CAGGCAGGAG TGAGCCTCAG
1101  GCCCCGTCCC TGCACCCCAC CCCTGCGTGC GCCTCTTGGT GATGCTGGGG
1151  TCTCACTAGC TTGAGGGGGC ACATGAAGAT AAGCCACAAA TGAAGAGAAA
1201  AGCCATGCCC ACCCCAGCCC CAGAGAAACC AATAAGAATC CTCTATTATT
1251  TTCACTATTC ATTTAGGTTT TTATACTCCA CCTCCTTTCA AAAAAGATTT
1301  AAGATGTACG ACATTACCGA ACACCTAAAA TAGAACCAGA GAAACGAAAG
1351  CCATTCCCAC AAAGTGAAGG AACAGTTTCC AAAACCCCTG CGAGGCAGAG
1401  TTAAGACCTG TGACGTGGGG AGCCTGGAAA CACCCGGCCC ACGCGTGCTG
1451  CAGATGTGGG GAGCCTGGAA ACACCCGGCC CACGCGTGCT GCAGATGTGG
1501  GGAGCCTGGA AACACCCGGC CCACGCGTGC TGCAGATGTG GGGAGTCTGG
1551  AAACACCCGG CCCACGCGTG CTGCAGATGT GGGGAGCCCG GAAACACCTG
1601  GCCCATGCCT GTTCAGTCCG TCATGGCGTT TGGTTTATGG TGGCCGATAC
1651  TAAGAGCCAC TGAGGTCTGG GTTGTGTAAG GGTTAGCTAC CACCCTATAA
1701  CCTCCCTACC AGGTAATAAG GAGACCAAGT CTTTGATCAA ATTCTGATTT
1751  TTTAACTAAA TTTTCATTAG AAAGCAAGCA CAAAATAAAA TCAAAACAAC
1801  CTTTACCTTC
```

Fig. 5 : C) SEQ ID NO: 7, nucleotide sequence of human ADARB2 sv3 cDNA

Length: 703 bp

```
  1  AGAAAAGAGA GCAGCATTTG TTAGCACACA TGGGCCTGTG GAGCCCAGTC
 51  CCAGGCAACA GTGTCCCCAG AGCCCCAGTG ATGGCTGGGT TGGGTCTGGG
101  GCACTCCCAG GGAGCTCCCC AAGGTGGAAC GTCCTGGGGC TGCAGGGCGC
151  GCTCCTGTCC CACTTCGTGG AGCCCGTGTA CCTGCAGAGC ATCGTGGTGG
201  GCAGCCTGCA CCACACGGGC CACCTCGCAC GCGTCATGAG CCACCGCATG
251  GAGGGTGTCG GCCAGCTGCC CGCCTCCTAC CGGCACAACC GGCCTCTCCT
301  CAGCGGCGTG AGTGACGCCG AGGCGCGCCA GCCGGGGAAG TCGCCCCCCT
351  TCAGCATGAA CTGGGTCGTG GGCAGCGCGG ACCTGGAGAT TATCAACGCC
401  ACCACTGGGC GGAGGAGCTG TGGGGGCCCA TCCCGGCTCT GCAAGCACGT
451  GCTGTCTGCA CGGTGGGCGC GGCTGTATGG CAGGCTGAGC ACACGGACAC
501  CCAGCCCTGG AGACACGCCC TCCATGTACT GTGAGGCCAA GCTGGGGGCG
551  CACACCTACC AGTCTGTGAA ACAGCAGCTG TTCAAGGCCT TTCAGAAGGC
601  TGGCCTGGGC ACCTGGGTGA GGAAACCACC GGAGCAGCAG CAGTTTCTAC
651  TGACTCTCTA GGCTGCGGGC TCCTGGCTGC TGGAGCTGAG CGGGACGCTG
701  GAG
```

Fig. 5 : D) SEQ ID NO: 8, nucleotide sequence of human ADARB2 sv4 cDNA

Length: 555 bp

```
  1  ATGAATGAGA GGGAGACACC CGGCCAGCTG GGCTTGGGGA ACGCGTCAGG
 51  CGTGAGTGAC GCCGAGGCGC GCCAGCCGGG GAAGTCGCCC CCCTTCAGCA
101  TGAACTGGGT CGTGGGCAGC GCGGACCTGG AGATTATCAA CGCCACCACT
151  GGGCGGAGGA GCTGTGGGGG CCCATCCCGG CTCTGCAAGC ACGTGCTGTC
201  TGCACGGTGG GCGCGGCTGT ATGGCAGGCT GAGCACACGG ACACCCAGCC
251  CTGGAGACAC GCCCTCCATG TACTGTGAGG CCAAGCTGGG GGCGCACACC
301  TACCAGTCTG TGAAACAGCA GCTGTTCAAG GCCTTTCAGA AGGCTGGCCT
351  GGGCACCTGG GTGAGGAAAC CACCGGAGCA GCAGCAGTTT CTACTGACTC
401  TCTAGGCTGC GGGCTCCTGG CTGCTGGAGC TGAGCGGGAC GCTGGAGGGA
451  TGGGACCGTG TCTGGGGGGC GACGTGGCGG GTCGGCCGGT TCCCTGCATT
501  CGTTTTACTT TGGTGTCCCA GAAACACGCG AGTGTGCAAT GTTTGGACGA
551  GCAAC
```

Fig. 6 : A) SEQ ID NO: 9, coding nucleotide sequence of human ADARB2 sv1 cDNA

Length: 2220 bp

```
   1 ATGGCCTCGG TCCTGGGGAG CGGCAGAGGG TCTGGAGGGC TGAGCAGTCA
  51 ACTCAAATGC AAGTCCAAGA GGAGGAGGAG GCGGAGGTCC AAGCGGAAAG
 101 ATAAAGTAAG CATATTGTCA ACCTTCCTCG CTCCTTTCAA GCACCTGAGT
 151 CCTGGCATCA CAAACACGGA GGATGACGAC ACCCTCAGTA CCAGCAGCGC
 201 GGAGGTGAAG GAGAACCGCA ACGTGGGCAA CCTGGCCGCG CGGCCACCGC
 251 CCTCCGGGGA CCGGGCCCGG GGCGGCGCGC CCGGCGCGAA GAGGAAGCGG
 301 CCGCTGGAGG AGGGGAATGG GGGCCACTTG TGCAAACTGC AGCTGGTCTG
 351 GAAGAAGCTG TCGTGGTCGG TGGCGCCCAA GAACGCGCTG GTGCAGCTGC
 401 ACGAGCTGAG GCCGGGCCTG CAGTACCGGA CAGTGTCGCA GACGGGCCCG
 451 GTGCATGCCC CGGTCTTCGC GGTAGCGGTG GAGGTGAACG GGCTCACGTT
 501 CGAGGGCACA GGCCCCACCA AGAAGAAGGC CAAGATGCGC GCGGCGGAGC
 551 TGGCACTCAG GTCCTTCGTG CAGTTCCCCA ACGCCTGCCA GGCGCACCTG
 601 GCCATGGGCG GGGGCCCGGG CCCCGGCACG GACTTCACCT CCGACCAGGC
 651 CGATTTCCCC GACACGCTCT TCCAGGAGTT CGAGCCCCCG GCGCCGCGCC
 701 CCGGACTCGC GGGAGGCCGC CCCGGGGACG CCGCGCTTCT GTCCGCGGCC
 751 TACGGGCGAC GGCGGCTGCT GTGCCGCGCG CTGGACCTGG TGGGCCCGAC
 801 CCCCGCCACC CCCGCGGCCC CGGGCGAGCG CAACCCCGTG GTGCTGCTGA
 851 ACCGCCTGCG CGCCGGGCTG CGCTACGTGT GTCTGGCAGA ACCGGCCGAG
 901 CGGCGCGCGC GGAGCTTCGT GATGGCCGTG AGCGTGGACG GCAGGACGTT
 951 CGAGGGCTCG GGGCGCAGCA AGAAGCTGGC CCGGGGTCAG GCCGCGCAGG
1001 CCGCACTGCA GGAGCTGTTC GACATCCAGA TGCCCGGCCA CGCGCCCGGC
1051 AGGGCCAGGA GGACGCCAAT GCCGCAGGAA TTCGCAGACT CCATATCCCA
1101 GCTGGTCACA CAGAAGTTCC GCGAGGTGAC GACGGACCTC ACGCCCATGC
1151 ACGCCCGCCA TAAAGCGCTG GCAGGAATCG TCATGACCAA AGGCCTGGAT
1201 GCTCGGCAGG CGCAGGTCGT GGCCCTGTCC TCGGGGACCA AGTGCATCAG
1251 CGGCGAGCAC CTCAGTGACC AGGGGCTGGT GGTGAATGAC TGCCACGCGG
1301 AGGTCGTGGC CCGGCGGGCG TTCCTGCACT TCCTCTACAC GCAGCTGGAG
1351 CTGCACCTGA GCAAGCGGCG CGAGGACTCA GAGCGATCGA TATTCGTGCG
1401 GTTAAAAGAA GGTGGCTACC GGCTGCGAGA GAACATCCTC TTCCATCTCT
1451 ACGTGAGCAC CTCCCCCTGT GGAGACGCAA GACTCCACTC TCCCTACGAG
1501 ATCACCACAG ACCTGCACAG CAGCAAACAC CTCGTCAGGA AGTTCCGCGG
1551 GCACCTGCGC ACCAAGATCG AGTCCGGGGA AGGGACGGTC CCCGTGCGTG
1601 GCCCCAGCGC AGTGCAGACC TGGGACGGCG TCCTGCTGGG GGAGCAGCTG
1651 ATCACCATGT CCTGCACGGA CAAGATCGCC AGGTGGAACG TCCTGGGGCT
1701 GCAGGGCGCG CTCCTGTCCC ACTTCGTGGA GCCCGTGTAC CTGCAGAGCA
1751 TCGTGGTGGG CAGCCTGCAC CACACGGGCC ACCTCGCACG CGTCATGAGC
1801 CACCGCATGG AGGGTGTCGG CCAGCTGCCC GCCTCCTACC GGCACAACCG
1851 GCCTCTCCTC AGCGGCGTGA GTGACGCCGA GGCGCGCCAG CCGGGGAAGT
1901 CGCCCCCCTT CAGCATGAAC TGGGTCGTGG GCAGCGCGGA CCTGGAGATT
1951 ATCAACGCCA CCACTGGGCG GAGGAGCTGT GGGGGCCCAT CCCGGCTCTG
2001 CAAGCACGTG CTGTCTGCAC GGTGGGCGCG GCTGTATGGC AGGCTGAGCA
2051 CACGGACACC CAGCCCTGGA GACACGCCCT CCATGTACTG TGAGGCCAAG
2101 CTGGGGGCGC ACACCTACCA GTCTGTGAAA CAGCAGCTGT TCAAGGCCTT
2151 TCAGAAGGCT GGCCTGGGCA CCTGGGTGAG GAAACCACCG GAGCAGCAGC
2201 AGTTTCTACT GACTCTCTAG
```

Fig. 6 : B) SEQ I D NO: 1 0,
coding nucleotide sequence
of human ADARB2 sv2 cDNA

Length: 747 bp

```
  1  ATGGGCCTGT GGAGCCCAGT CCCAGGCAAC AGTGTCCCCA GAGCCCCAGT
 51  GATGGCTGGG TTGGGTCTGG GGCACTCCCA GGGAGCTCCC CAAGGTCCGC
101  ACGATGGGAA GACTGACAGG TCCTCAGGAC CAGGCCGCAG ACGGACAGAG
151  AGGACCAGGC TCCCTGGGCT CCTGGGTGGG GCTCCGCTGA CTGCTGGCTC
201  TCCCCACAGG TGGAACGTCC TGGGGCTGCA GGGCGCGCTC CTGTCCCACT
251  TCGTGGAGCC CGTGTACCTG CAGAGCATCG TGGTGGGCAG CCTGCACCAC
301  ACGGGCCACC TCGCACGCGT CATGAGCCAC CGCATGGAGG GTGTCGGCCA
351  GCTGCCCGCC TCCTACCGGC ACAACCGGCC TCTCCTCAGC GGCGTGAGTG
401  ACGCCGAGGC GCGCCAGCCG GGGAAGTCGC CCCCCTTCAG CATGAACTGG
451  GTCGTGGGCA GCGCGGACCT GGAGATTATC AACGCCACCA CTGGGCGGAG
501  GAGCTGTGGG GGCCCATCCC GGCTCTGCAA GCACGTGCTG TCTGCACGGT
551  GGGCGCGGCT GTATGGCAGG CTGAGCACAC GGACACCCAG CCCTGGAGAC
601  ACGCCCTCCA TGTACTGTGA GGCCAAGCTG GGGGCGCACA CCTACCAGTC
651  TGTGAAACAG CAGCTGTTCA AGGCCTTTCA GAAGGCTGGC CTGGGCACCT
701  GGGTGAGGAA ACCACCGGAG CAGCAGCAGT TTCTACTGAC TCTCTAG
```

Fig. 6 : C) SEQ ID NO: 11, coding nucleotide sequence of human ADARB2 sv3 cDNA

Length: 426 bp

```
  1  ATGAGCCACC GCATGGAGGG TGTCGGCCAG CTGCCCGCCT CCTACCGGCA
 51  CAACCGGCCT CTCCTCAGCG GCGTGAGTGA CGCCGAGGCG CGCCAGCCGG
101  GGAAGTCGCC CCCCTTCAGC ATGAACTGGG TCGTGGGCAG CGCGGACCTG
151  GAGATTATCA ACGCCACCAC TGGGCGGAGG AGCTGTGGGG GCCCATCCCG
201  GCTCTGCAAG CACGTGCTGT CTGCACGGTG GGCGCGGCTG TATGGCAGGC
251  TGAGCACACG GACACCCAGC CCTGGAGACA CGCCCTCCAT GTACTGTGAG
301  GCCAAGCTGG GGGCGCACAC CTACCAGTCT GTGAAACAGC AGCTGTTCAA
351  GGCCTTTCAG AAGGCTGGCC TGGGCACCTG GGTGAGGAAA CCACCGGAGC
401  AGCAGCAGTT TCTACTGACT CTCTAG
```

Fig. 6 : D) SEQ ID NO: 12, coding nucleotide sequence of human ADARB2 sv4 cDNA

Length: 405 bp

```
  1  ATGAATGAGA GGGAGACACC CGGCCAGCTG GGCTTGGGGA ACGCGTCAGG
 51  CGTGAGTGAC GCCGAGGCGC GCCAGCCGGG GAAGTCGCCC CCCTTCAGCA
101  TGAACTGGGT CGTGGGCAGC GCGGACCTGG AGATTATCAA CGCCACCACT
151  GGGCGGAGGA GCTGTGGGGG CCCATCCCGG CTCTGCAAGC ACGTGCTGTC
201  TGCACGGTGG GCGCGGCTGT ATGGCAGGCT GAGCACACGG ACACCCAGCC
251  CTGGAGACAC GCCCTCCATG TACTGTGAGG CCAAGCTGGG GGCGCACACC
301  TACCAGTCTG TGAAACAGCA GCTGTTCAAG GCCTTTCAGA AGGCTGGCCT
351  GGGCACCTGG GTGAGGAAAC CACCGGAGCA GCAGCAGTTT CTACTGACTC
401  TCTAG
```

Fig. 7: Alignment of ADARB2 RT-PCR primers SEQ ID NO: 13 and SEQ ID NO: 14 with human ADARB2 cDNA, SEQ ID NO: 5

```
SEQ ID NO:13 (PRIMER A)        1 GAGTGTGCAATGTTTGGACGA 21
                                 |||||||||||||||||||||
SEQ ID NO:5                 2679 GAGTGTGCAATGTTTGGACGA 2699


SEQ ID NO:14 (PRIMER B)       19 GCACACGCACCGTTGAGTT 1
                                 |||||||||||||||||||
SEQ ID NO:5                 2772 GCACACGCACCGTTGAGTT 2790
```

Fig. 8: Schematic alignment of SEQ ID NO: 5, SEQ ID NO: 9, and RT-PCR primer sequences of ADARB2

```
SEQ ID NO:13                                      +>                  2671-2691
SEQ ID NO:14                                      <+                  2782-2764
SEQ ID NO:9        +------------------------------->                  327-2546
SEQ ID NO:5    +--------------------------------------------------->  1-3606

               |-----|-----|-----|-----|-----|-----|-----|-----|-----|-----|
               0    400   800  1200  1600  2000  2400  2800  3200  3600  4000
```

Legend:
```
+      5'-end of sequence
>      3'-end of sequence
---    sequence identical to ADARB2 mRNA
```

Fig. 9: Westernblot analysis of ADARB2 antibody sc-10014

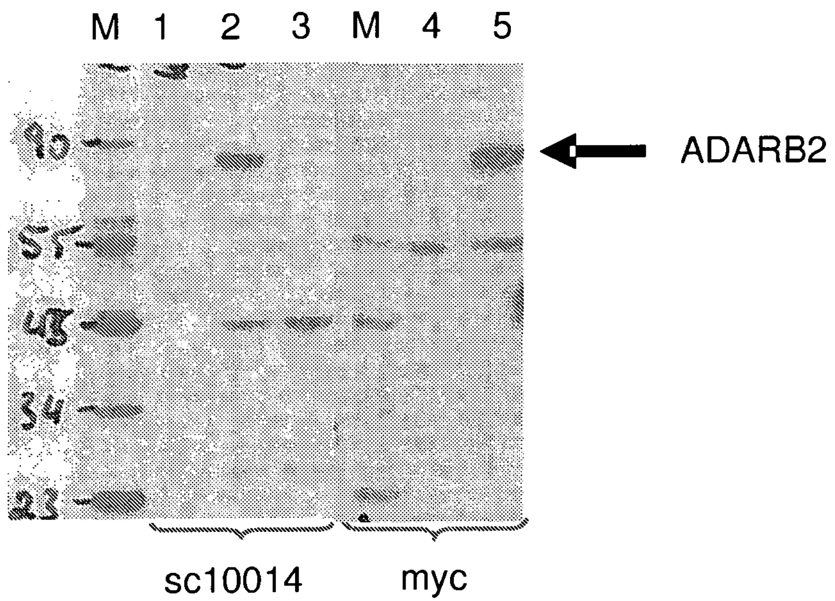


Legend: M: molecular weight marker

Lane 1 – 3 probed with sc-10014 (1:200):
Human brain extract (1), CHO+ADARB2-myc (2), CHO negative control (3)

Lane 4 – 5 probed with anti-myc antibody (1:3000):
CHO negative control (4), CHO+ADARB2-myc (5)

Fig. 10: A) Expression of ADARB2 protein is increased in the cerebral cortex of AD patients

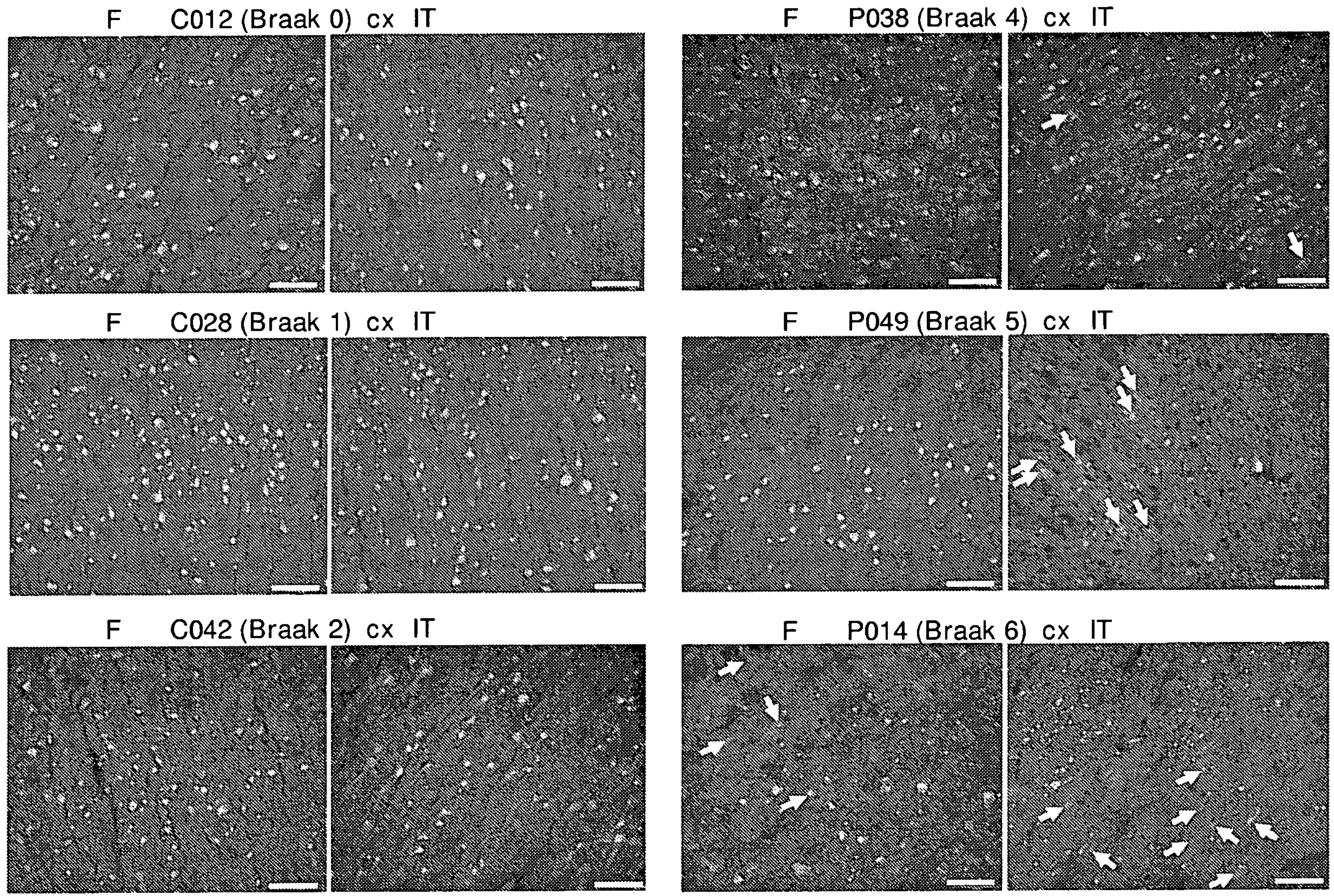


bar=100 μm

Figure 10: B) Expression of ADARB2 protein is increased in the cerebral white matter of AD patients

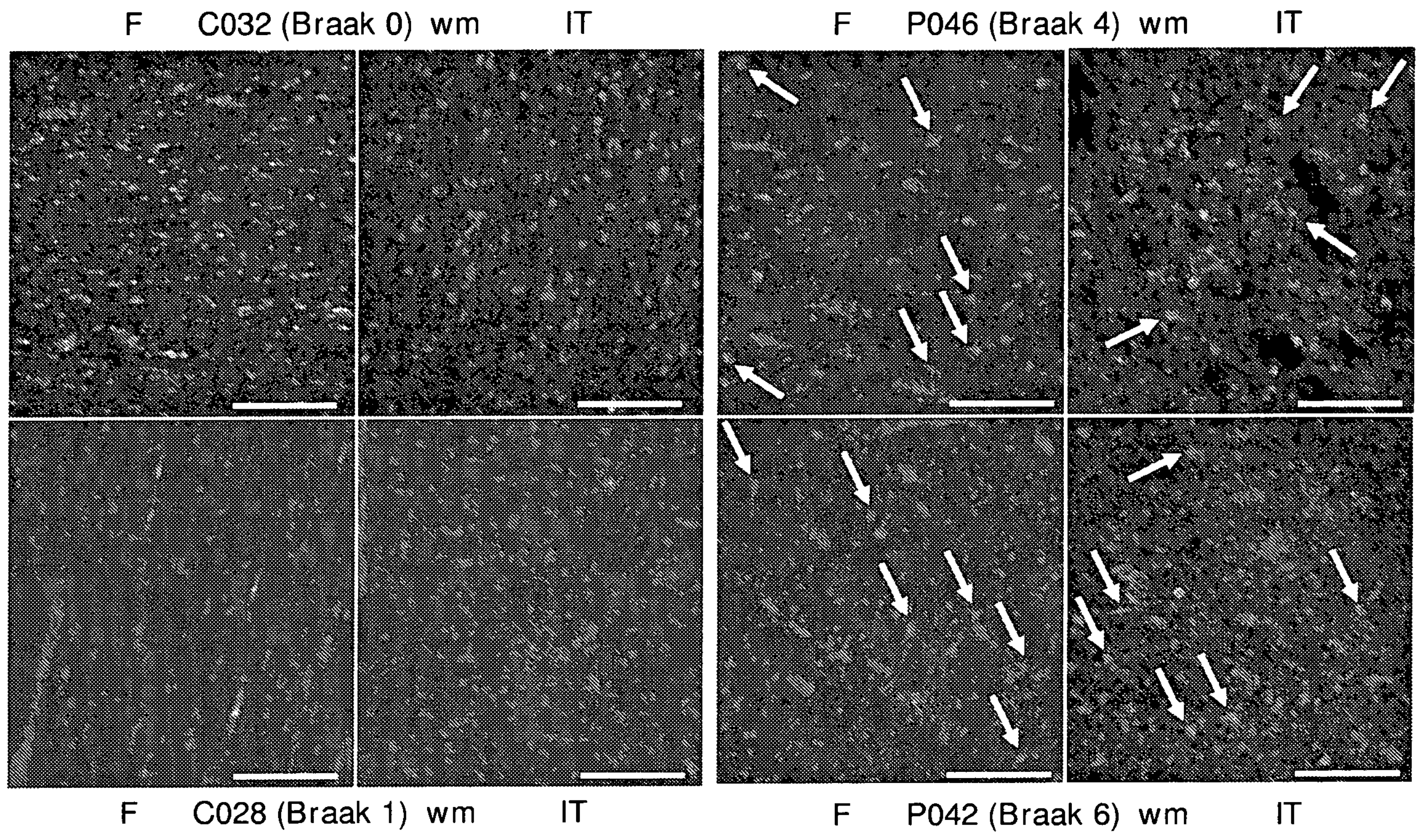


bar=100 μm

Figure 11: Westernblot analysis of inducible expression of ADARB2 in H4APPsw-tissue culture cells

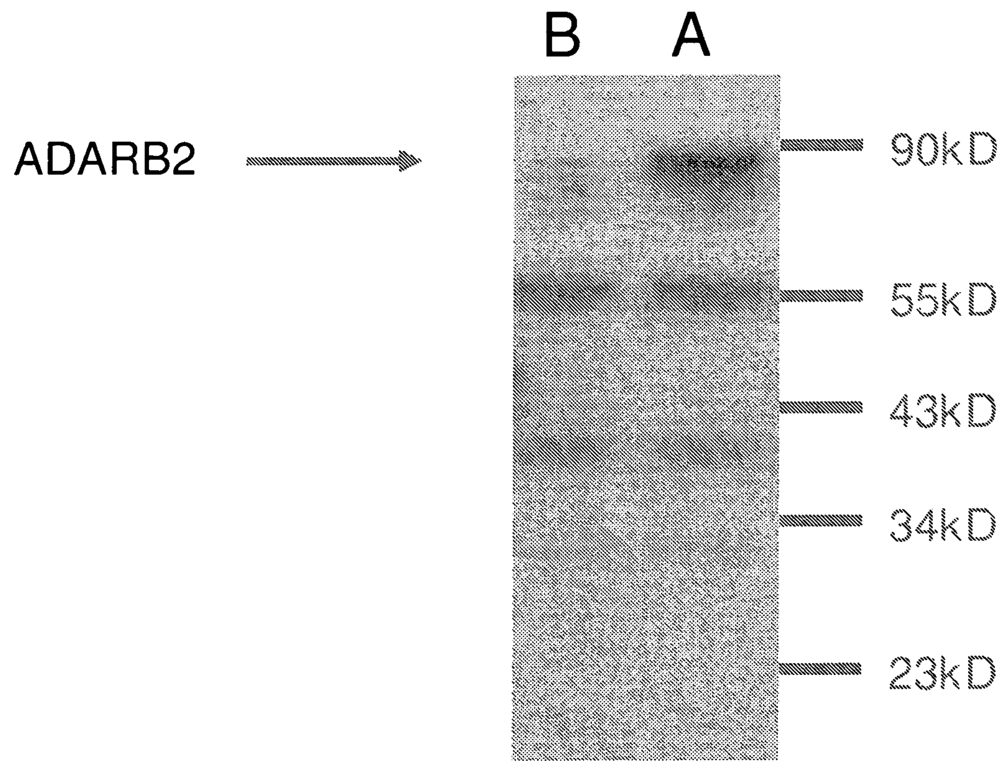


A: H4 APPsw-ADARB2-myc +tetracycline (induced)
B: H4 APPsw-ADARB2-myc –tetracycline (control)

Figure 12: Immunofluorescence analysis of inducible ADARB2 expression in H4APPsw-cells

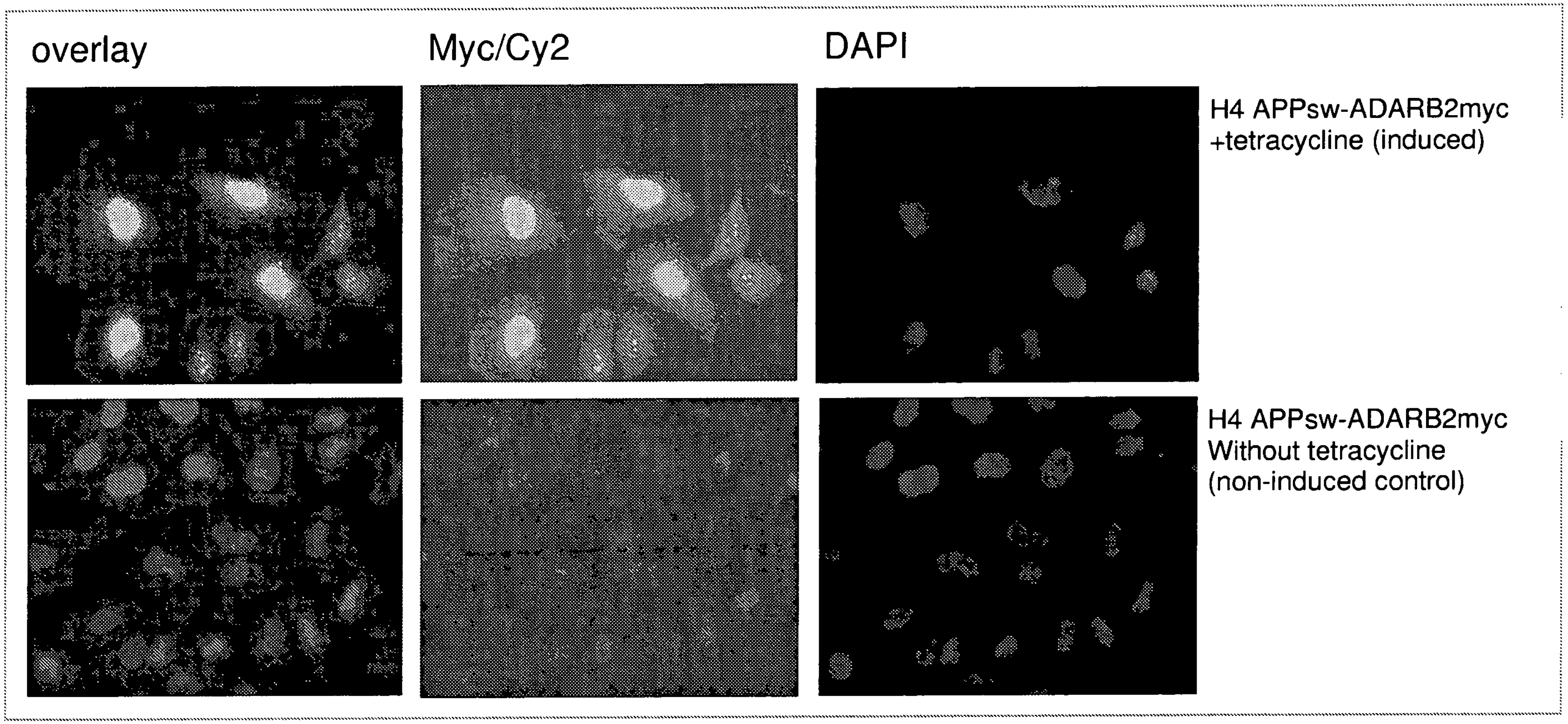

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5763174 A
- WO 0214543 A
- WO 0052451 A
- WO 020122 A
- WO 9613744 A
- WO 9816814 A
- WO 9823942 A
- WO 9917086 A
- WO 9934195 A
- WO 0066985 A
- WO 0159436 A
- WO 0159416 A
- US 6150173 A


### Non-patent literature cited in the description


- **Vickers et al.** *Progress in Neurobiology,* 2000, vol. 60, 139-165
- **Walsh ; Selkoe.** *Neuron,* 2004, vol. 44, 181-193
- **Selkoe ; Kopan.** *Annu Rev Neurosci,* 2003, vol. 26, 565-597
- **Ling et al.** *Int J Biochem Cell Biol,* 2003, vol. 35, 1505-1535
- **Braak ; Braak.** *J Neural Transm,* 1998, vol. 53, 127-140
- **Johnson ; Jenkins.** *J Alzheimers Dis,* 1996, vol. 1, 38-58
- **Johnson ; Hartigan.** *J Alzheimers Dis,* 1999, vol. 1, 329-351
- **Schmitt et al.** *Neurology,* 2000, vol. 55, 370-376
- **Terry et al.** *Annals of Neurology,* 1981, vol. 10, 184-92
- **Strittmatter et al.** *Proc Natl Acad Sci USA,* 1993, vol. 90, 1977-81
- **Roses.** *Ann NY Acad Sci,* 1998, vol. 855, 738-43
- **Seeburg.** *Neuron,* 2002, vol. 35, 17-20
- **Maas et al.** *J. Biol. Chem.,* 2003, vol. 278, 1391-1394
- **Reenan.** *Trends in Genetics,* 2001, vol. 17, 53-56
- **Kwak et al.** *J. Mol. Med.,* 2005, vol. 83, 110-120
- **Higuchi et al.** *Nature,* 2000, vol. 406, 78-81
- **Wang et al.** *Science,* 2000, vol. 290, 1765-1768
- **Melcher et al.** *J. Biol. Chem.,* 1996, vol. 271, 31795-31798
- **Chen et al.** *RNA,* 2000, vol. 6, 755-767
- **Altschul et al.** *J. Mol. Biol.,* 1990, vol. 215, 403-410
- **Altschul et al.** *Nucleic Acids Res.,* 1997, vol. 25, 3389-3402
- **Devereux et al.** *Nucleic Acids Res.,* 1984, vol. 12, 387
- **Pearson ; Lipman.** *Proc. Natl. Acad. Sci. USA,* 1988, vol. 85, 2444-2448
- **Wilbur ; Lipman.** *SIAM J. Appl. Math.,* 1984, vol. 44, 557-567
- **Needleman ; Wunsch.** *J. Mol. Biol.,* vol. 48, 443-453
- **Iqbal ; Swaab ; Winblad ; Wisniewski.** Alzheimer's Disease and Related Disorders (Etiology, Pathogenesis and Therapeutics). Wiley & Sons, 1999
- **Scinto ; Daffner.** Early Diagnosis of Alzheimer's Disease. Humana Press, 2000
- **Mayeux ; Christen.** Epidemiology of Alzheimer's Disease: From Gene to Prevention. Springer Press, 1999
- **Younkin ; Tanzi ; Christen.** Presenilins and Alzheimer's Disease. Springer Press, 1998
- **Braak ; Braak.** *Acta Neuropathology,* 1991, vol. 82, 239-259
- **Bancher et al.** *Neuroscience Letters,* 1993, vol. 162, 179-182
- **Gold et al.** *Acta Neuropathol,* 2000, vol. 99, 579-582
- **R6ssler et al.** *Acta Neuropathol,* 2002, vol. 103, 363-369
- **Giannakopoulos et al.** *Neurology,* 2003, vol. 60, 1495-1500
- **Bennett et al.** *Arch Neurol,* 2004, vol. 61, 378-384
- **Metsaars et al.** *Neurobiol Aging,* 2003, vol. 24, 563-572
- **Terry et al.** *Annals of Neurology,* 1981, vol. 10, 184-192

- **Sambrook ; Russell.** Molecular Cloning: A Laboratory Manual. Cold Spring Harbor Laboratory Press, 2001
- **Schena M.** Microarray Biochip Technology. Eaton Publishing, 2000
- **Harlow ; Lane.** Antibodies: A Laboratory Manual. Cold Spring Harbor Laboratory Press, 1999
- **Edwards R.** Immunodiagnostics: A Practical Approach. Oxford University Press, 1999
- **Schena M.** *Microarray Biochip Technology,* 2000
- **Behr.** *Acc Chem Res,* 1993, vol. 26, 274-278
- **Mulligan.** *Science,* 1993, vol. 260, 926-931
- **Wolff.** *Curr Opin Neurobiol,* 1993, vol. 3, 743-748
- **Gillespie.** *DM&P,* 1992, vol. 5, 389-395
- **Agrawal ; Akhtar.** *Trends Biotechnol,* 1995, vol. 13, 197-199
- **Crooke.** *Biotechnology,* 1992, vol. 10, 882-6
- **Barinaga.** *Science,* 1993, vol. 262, 1512-1514
- **Wickstrom.** *Trends Biotechnol,* 1992, vol. 10, 281-287
- **Hannon.** *Nature,* 2002, vol. 418, 244-251
- **Mc Celland ; Pardee.** Expression Genetics: Accelerated and High-Throughput Methods. Eaton Publishing, 1999
- **Lanza et al.** *Nature Medicine,* 1999, vol. 9, 975-977
- **Peterson DA.** *Curr. Opin. Pharmacol.,* 2002, vol. 2, 34-42
- **Colman A.** *Drug Discovery World,* 2001, vol. 7, 66-71
- **Capecchi.** *Science,* 1989, vol. 244, 1288-1292
- **Hogan et al.** Manipulating the Mouse Embryo: A Laboratory Manual. Cold Spring Harbor Laboratory Press, 1994
- **Jackson ; Abbott.** Mouse Genetics and Transgenics: A Practical Approach. Oxford University Press, 1999
- **Schwarz et al.** *Biochemistry,* 1999, vol. 38, 9456-9464
- **Krivosheev ; Usanov.** *Biochemistry-Moscow,* 1997, vol. 62, 1064-1073
- **Harlow et al.** Antibodies, A Laboratory Manual. Cold Spring Harbor Laboratory Press, 1988
- **Dubel ; Breitling.** Recombinant Antibodies. Wiley-Liss, 1999
- **Harlow ; Lane.** Using Antibodies: A Laboratory Manual. Cold Spring Harbor Laboratory Press, 1999
- **Edwards R.** Immunodiagnostics: A Practical Approach. Oxford University Press, 1999
- **Sachs L.** Statistische Methoden: Planung und Auswertung. 1988, 60